(19) **Europäisches Patentamt / European Patent Office / Office européen des brevets**

(11) **EP 4 206 223 A1**

(12) # EUROPEAN PATENT APPLICATION
published in accordance with Art. 153(4) EPC

(43) Date of publication:
**05.07.2023 Bulletin 2023/27**

(21) Application number: **21861697.7**

(22) Date of filing: **27.08.2021**

(51) International Patent Classification (IPC):
*C07K 16/00* (2006.01)      *C07K 16/46* (2006.01)
*C12N 15/13* (2006.01)

(52) Cooperative Patent Classification (CPC):
**C07K 16/00; C07K 16/46**

(86) International application number:
**PCT/JP2021/031448**

(87) International publication number:
**WO 2022/045276 (03.03.2022 Gazette 2022/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.08.2020 PCT/JP2020/032547**

(71) Applicant: **CHUGAI SEIYAKU KABUSHIKI KAISHA
Tokyo 115-8543 (JP)**

(72) Inventors:
• **KATADA, Hitoshi
  Gotemba-shi, Shizuoka 412-8513 (JP)**
• **TATSUMI, Kanako
  Gotemba-shi, Shizuoka 412-8513 (JP)**

(74) Representative: **Vossius & Partner
Patentanwälte Rechtsanwälte mbB
Siebertstraße 3
81675 München (DE)**

Remarks:
The complete document including Reference
Table(s) and the Sequence Listing(s) can be
downloaded from the EPO website

(54) **HETERODIMER FC POLYPEPTIDE**

(57)    In one non-limiting embodiment, polypeptides comprising a variant Fc region which contains amino acid alterations in a parent Fc region, and methods for producing such polypeptides, are provided.

FIG. 1

EP 4 206 223 A1

**Description**

[Technical Field]

In one non-limiting embodiment, the present disclosure relates to polypeptides comprising a variant Fc region which comprises amino acid alterations in a parent Fc region, and methods for producing such polypeptides.

[Background Art]

Antibodies are drawing attention as pharmaceuticals because of their high stability in the blood and less side effects (NPL 1 and NPL 2). Most of the currently marketed antibody pharmaceuticals are human IgG1 subclass antibodies. Many studies have so far been carried out on the effector functions of IgG class antibodies, namely, antibody-dependent cellular cytotoxicity (hereinafter denoted as ADCC) and complement-dependent cytotoxicity (hereinafter denoted as CDC). It has been reported that, among the human IgG class, IgG1 subclass antibodies have the highest ADCC activity and CDC activity (NPL 3). In addition, antibody-dependent cell-mediated phagocytosis (ADCP), which is phagocytosis of target cells mediated by IgG class antibodies, has also been shown as one of the antibody effector functions (NPL 4 and NPL 5).

In order for IgG antibodies to exert ADCC, CDC, and ADCP, the antibody Fc region needs to bind to an antibody receptor (hereinafter denoted as FcγR) present on the surface of effector cells such as killer cells, natural killer cells, and activated macrophages, and to various complement components. In humans, the FcγR protein family reportedly has isoforms FcγRIa, FcγRIIa, FcγRIIb, FcγRIIIa, and FcγRIIIb, and allotypes of each have also been reported (NPL 6).

Enhancement of cytotoxic effector functions such as ADCC, ADCP, and CDC is attracting attention as a promising means to strengthen the antitumor effect of antibodies. The importance of FcγR-mediated effector functions for the antitumor effect of antibodies has been reported using mouse models (NPL 7 and NPL 8). In addition, a correlation was observed between clinical results in humans and the FcγRIIIa high-affinity polymorphism allotype (V158) and low-affinity polymorphism allotype (F158) (NPL 9). Similarly, it has also been shown that clinical effects vary with FcγRIIa allotypes (H131 and R131) (NPL 10). These reports indicate that antibodies possessing an Fc region with optimized binding to a particular FcγR will mediate more potent effector functions and thereby exert effective antitumor effects.

The balance of binding activities of antibody toward the activating receptors constituted by FcγRIa, FcγRIIa, FcγRIIIa, and FcγRIIIb, and toward the inhibitory receptor constituted by FcγRIIb is an important element in optimizing the antibody effector functions. By using an Fc region with enhanced binding activity to activating receptors and reduced binding activity to inhibitory receptors, it may be possible to impart optimum effector functions to an antibody (NPL 11). For the binding between the Fc region and FcγRs, several amino acid residues within the antibody hinge region and CH2 domain, and the sugar chain attached to Asn at position 297 according to EU numbering, which is bound to the CH2 domain, have been shown to be important (NPL 12, NPL 13, and NPL 14). With a focus on this binding site, Fc region variants with various FcγR-binding properties have been studied so far, and Fc region variants with higher binding activity to activating FcγRs have been obtained (PTL 1 and PTL 2). For example, Lazar *et al.* substituted Ser at position 239, Ala at position 330, and Ile at position 332 according to EU numbering in human IgG1 with Asp, Leu, and Glu, respectively, and thereby successfully increased its binding to human FcγRIIIa(V158) up to about 370 times (NPL 15 and PTL 2). Shinkawa *et al.* succeeded in increasing the binding to FcγRIIIa up to about 100 times by deleting fucose in the sugar chain attached to Asn at EU numbering position 297 (NPL 16). These methods introduce the same alterations, or the same sugar chain modifications, into both of the two H chain Fc regions of an antibody. In the meantime, it has been reported that although the antibody Fc is a homodimer, it binds to FcγRs at 1:1, recognizing FcγRs asymmetrically via the lower hinge and the CH2 domain (NPL 17). In view of fact that the Fc region asymmetrically interacts with FcγRs, it may be possible to optimize the IgG-FcγR interaction more finely by introducing different modifications into each H chain. Based on this idea, methods for optimizing the antibody-FcγR interaction by modifying the Fc region of each H chain differently so that Fc is asymmetrically modified have also been reported (PTL 3, PTL 4, PTL 5, and PTL 6). In fact, asymmetrical modification of the Fc region yielded variants with higher ADCC activity than afucosylated antibodies, which are existing ADCC-enhanced antibodies (PTL 5 and PTL 6).

In addition to ADCC activity, ADCP activity is also an important effector function of antibody, and has been reported to contribute to antitumor effects (NPL 18). ADCP activity can be enhanced by inhibiting the "Don't eat me" signal, as represented by CD47 (NPL 18), and also be enhanced by strengthening the FcγRIIa-binding ability (NPL 19). However, FcγRIIa, an activating FcγR, and inhibitory FcγRIIb share a very high homology in the amino acid sequence of the extracellular region, and therefore selective enhancement of the FcγRIIa-binding ability is difficult (NPL 20). Accordingly, when the FcγRIIa-binding ability is enhanced, the binding activity to FcγRIIb, an inhibitory receptor, is also likely to be enhanced, thereby weakening the effector functions. In fact, variants with greatly improved FcγRIIa-binding ability also had stronger FcγRIIb-binding ability than native IgG1 (PTL 5 and PTL 6). Thus, in order to exhibit high ADCC/ADCP activity, it is preferable to enhance the binding to FcγRIIIa and/or FcγRIIa as much as possible without enhancing the binding to FcγRIIb; however, no such variant has been reported.

[Citation List]

[Patent Literature]

[PTL 1] WO 2000/042072
[PTL 2] WO 2006/019447
[PTL 3] WO 2012/058768
[PTL 4] WO 2012/125850
[PTL 5] WO 2013/002362
[PTL 6] WO 2014/104165

[Non-Patent Literature]

[NPL 1] Nature Biotechnology, 23, 1073 - 1078 (2005)
[NPL 2] Eur. J. Pharm. Biopharm, 59(3), 389-96 (2005)
[NPL 3] Chemical Immunology, 65, 88 (1997)
[NPL 4] Cancer Res., 68, 8049-8057 (2008)
[NPL 5] Blood, 113, 3735-3743 (2009)
[NPL 6] Immunol. Lett. 82, 57-65 (2002)
[NPL 7] Pro. Nat. Acad. Sci. 95:652-656 (1998)
[NPL 8] Nature Medicine, 6: 443-446 (2000)
[NPL 9] Blood, 99:754-758 (2002)
[NPL 10] Ann. Oncol., 22: 1302-1307 (2011)
[NPL 11] Science, 310, 1510-1512 (2005)
[NPL 12] Chemical Immunology, 65, 88 (1997)
[NPL 13] Eur. J. Immunol. 23, 1098 (1993)
[NPL 14] Immunology, 86, 319 (1995)
[NPL 15] Pro. Nat. Acad. Sci. 103, 4005-4010 (2006)
[NPL 16] J. Biol. Chem., 278, 3466-3473(2003)
[NPL 17] J. Biol. Chem., 276: 16469-16477, (2001)
[NPL 18] JCI Insight, 4: e131882, (2019)
[NPL 19] Mol. Cancer Ther., 7: 2517-2527, (2008)
[NPL 20] Protein Eng. Des. Sel., 26, 589-598, (2013)

[Summary of Invention]

[Technical Problem]

The invention of the present disclosure was made in view of these circumstances. In one aspect, an objective of the invention of the present disclosure is to provide polypeptides with improved Fc region functions (e.g. FcγR-binding ability, ADCC activity, and ADCP activity) as compared to Fc region-containing polypeptides that are conventional technology, and methods for producing such polypeptides.

[Solution to Problem]

In one non-limiting embodiment, the present disclosure provides the following:

[1] A polypeptide which comprises a variant Fc region comprising amino acid alterations in a parent Fc region, wherein the parent Fc region is composed of two polypeptide chains, and wherein the variant Fc region comprises amino acid alterations at the following positions:

(i) positions 234, 235, 236, 239, 268, 270, and 298 according to EU numbering in the first polypeptide of the parent Fc region; and
(ii) positions 270, 298, 326, and 334 according to EU numbering in the second polypeptide of the parent Fc region.

[2] The polypeptide of [1], wherein the variant Fc region further comprises an amino acid alteration at position 326 according to EU numbering in the first polypeptide of the parent Fc region.
[3] The polypeptide of [1] or [2], wherein the variant Fc region further comprises an amino acid alteration at position 236 according to EU numbering in the second polypeptide of the parent Fc region.
[4] A polypeptide which comprises a variant Fc region comprising amino acid alterations in a parent Fc region, wherein the parent Fc region is composed of two polypeptide chains, and wherein the variant Fc region comprises amino acid alterations at the following positions:

(i) positions 234, 235, 236, 239, 268, 270, 298, and 326 according to EU numbering in the first polypeptide of the parent Fc region; and
(ii) positions 236, 270, 298, 326, and 334 according to EU numbering in the second polypeptide of the parent Fc region.

[5] The polypeptide of any one of [1] to [4], wherein the variant Fc region further comprises an amino acid alteration at position 332 according to EU numbering in the first polypeptide of the parent Fc region.
[6] The polypeptide of any one of [1] to [5], wherein the variant Fc region further comprises an amino acid alteration at position 330 according to EU numbering in the first polypeptide of the parent Fc region.
[7] The polypeptide of any one of [1] to [6], wherein the variant Fc region further comprises an amino acid alteration at position 332 according to EU numbering in the second polypeptide of the parent Fc region.
[8] The polypeptide of any one of [1] to [7], wherein the variant Fc region further comprises an amino acid alteration at position 330 according to EU numbering in the second polypeptide of the parent Fc region.
[9] A polypeptide which comprises a variant Fc region comprising amino acid alterations in a parent Fc region, wherein the parent Fc region is composed of two polypeptide chains, and wherein the variant Fc region comprises amino acid alterations at the following positions:

(i) positions 234, 235, 236, 239, 268, 270, 298, 330, and 332 according to EU numbering in the first polypeptide of the parent Fc region; and
(ii) positions 236, 270, 298, 326, 330, 332, and 334 according to EU numbering in the second polypeptide of the parent Fc region.

[10] The polypeptide of any one of [1] to [9], wherein the variant Fc region further comprises amino acid alterations at positions 250 and 307 according to EU numbering in the first polypeptide of the parent Fc region.
[11] The polypeptide of any one of [1] to [10], wherein the variant Fc region further comprises amino acid alterations at positions 250 and 307 according to EU numbering in the second polypeptide of the parent Fc region.
[12] A polypeptide which comprises a variant Fc region comprising amino acid alterations in a parent Fc region, wherein the parent Fc region is composed of two polypeptide chains, and wherein the variant Fc region comprises amino acid alterations at the following positions:

(i) positions 234, 235, 236, 239, 250, 268, 270, 298, and 307 according to EU numbering in the first polypeptide of the parent Fc region; and
(ii) positions 250, 270, 298, 307, 326, and 334 according to EU numbering in the second polypeptide of the parent Fc region.

[13] A polypeptide which comprises a variant Fc region comprising amino acid alterations in a parent Fc region, wherein the parent Fc region is composed of two polypeptide chains, and wherein the variant Fc region comprises amino acid alterations at the following positions:

(i) positions 234, 235, 236, 239, 250, 268, 270, 298, 307, and 326 according to EU numbering in the first polypeptide of the parent Fc region; and
(ii) positions 236, 250, 270, 298, 307, 326, and 334 according to EU numbering in the second polypeptide of the parent Fc region.

[14] A polypeptide which comprises a variant Fc region comprising amino acid alterations in a parent Fc region, wherein the parent Fc region is composed of two polypeptide chains, and wherein the variant Fc region comprises amino acid alterations at the following positions:

(i) positions 234, 235, 236, 239, 250, 268, 270, 298, 307, 330, and 332 according to EU numbering in the first polypeptide of the parent Fc region; and
(ii) positions 236, 250, 270, 298, 307, 326, 330, 332, and 334 according to EU numbering in the second polypeptide of the parent Fc region.

[15] The polypeptide of any one of [1] to [14], which comprises at least one amino acid alteration selected from the following amino acid alterations:

(i) Tyr or Phe at position 234, Gin or Tyr at position 235, Trp at position 236, Met at position 239, Val at position 250, Asp at position 268, Glu at position 270, Ala at position 298, Pro at position 307, Asp at position 326, Met at position 330, and Glu at position 332 according to EU numbering in the first polypeptide of the parent Fc region; and
(ii) Ala at position 236, Val at position 250, Glu at position 270, Ala at position 298, Pro at position 307, Asp at position 326, Met or Lys at position 330, Asp or Glu at position 332, and Glu at position 334 according to EU numbering in the second polypeptide of the parent Fc region.

[16] The polypeptide of any one of [1] to [15], wherein the variant Fc region further comprises any of the amino acid alterations of (a) to (f) below:

(a) Lys at position 356 according to EU numbering in the first polypeptide of the parent Fc region, and Glu at position 439 according to EU numbering in the second polypeptide of the parent Fc region;
(b) Glu at position 439 according to EU numbering in the first polypeptide of the parent Fc region, and Lys at position 356 according to EU numbering in the second polypeptide of the parent Fc region;
(c) Trp at position 366 according to EU numbering in the first polypeptide of the parent Fc region, and Ser at position 366, Ala at position 368, and Val at position 407 according to EU numbering in the second polypeptide of the parent Fc region;
(d) Ser at position 366, Ala at position 368, and Val at position 407 according to EU numbering in the first polypeptide of the parent Fc region, and Trp at position 366 according to EU numbering in the second polypeptide of the parent Fc region;
(e) Cys at position 349 and Trp at position 366 according to EU numbering in the first polypeptide of the parent Fc region, and Cys at position 356, Ser at position 366, Ala at position 368, and Val at position 407 according to EU numbering in the second polypeptide of the parent Fc region;
(f) Cys at position 356, Ser at position 366, Ala at position 368, and Val at position 407 according to EU numbering in the first polypeptide of the parent Fc region, and Cys at position 349 and Trp at position 366 according to EU numbering in the second polypeptide of the parent Fc region.

[17] The polypeptide of any one of [1] to [16], wherein the variant Fc region further comprises any of the amino acid alterations of (a) to (d) below in the first polypeptide and/or the second polypeptide of the parent Fc region:

(a) Ala at position 434 according to EU numbering;
(b) Ala at position 434, Thr at position 436, Arg at position 438, and Glu at position 440 according to EU numbering;
(c) Leu at position 428, Ala at position 434, Thr at position 436, Arg at position 438, and Glu at position 440 according to EU numbering; and
(d) Leu at position 428, Ala at position 434, Arg at position 438, and Glu at position 440 according to EU numbering.

[18] The polypeptide of any one of [1] to [17], wherein binding activity to at least one Fcγ receptor selected from the group consisting of FcγRIa, FcγRIIa, FcγRIIb, and FcγRIIIa is enhanced in the variant Fc region as compared to the parent Fc region.
[19] The polypeptide of [18], wherein binding activity to FcγRIIa and FcγRIIIa is enhanced in the variant Fc region as compared to the parent Fc region.
[20] The polypeptide of any one of [1] to [19], wherein selectivity between an activating Fcγ receptor and an inhibitory Fcγ receptor is improved in the variant Fc region as compared to the parent Fc region.

[20-2] The polypeptide of any one of [1] to [19], wherein binding activity to an activating Fcγ receptor is selectively enhanced as compared to binding activity to an inhibitory Fcγ receptor in the variant Fc region, as compared to the parent Fc region.
[20-3] The polypeptide of any one of [1] to [19], wherein a ratio of binding activity to an activating Fcγ receptor to binding activity to an inhibitory Fcγ receptor (A/I ratio) is higher in the variant Fc region than the parent Fc region.

[20-4] The polypeptide of [20-3], wherein the ratio (A/I ratio) in the polypeptide comprising the variant Fc region is 1.1 times or more, 1.2 times or more, 1.3 times or more, 1.4 times or more, 1.5 times or more, 1.6 times or more, 1.7 times or more, 1.8 times or more, 1.9 times or more, 2 times or more, 3 times or more, 4 times or more, 5 times or more, 6 times or more, 7 times or more, 8 times or more, 9 times or more, 10 times or more, 20 times or more, 30 times or more, 40 times or more, 50 times or more, 60 times or more, 70 times or more, 80 times or more, 90 times or more, 100 times or more, 200 times or more, 300 times or more, 400 times or more, 500 times or more, 600 times or more, 700 times or more, 800 times or more, 900 times or more, 1000 times or more, 2000 times or more, 3000 times or more, 4000 times or more, 5000 times or more, 6000 times or more, 7000 times or more, 8000 times or more, 9000 times or more, or 10000 times or more higher than that in a polypeptide comprising the parent Fc region.

[20-5] The polypeptide of [20-3], wherein the ratio (All ratio) in the polypeptide comprising the variant Fc region has a value of 10 or higher, 20 or higher, 30 or higher, 40 or higher, 50 or higher, 60 or higher, 70 or higher, 80 or higher, 90 or higher, 100 or higher, 200 or higher, 300 or higher, 400 or higher, 500 or higher, 600 or higher, 700 or higher, 800 or higher, 900 or higher, 1000 or higher, 2000 or higher, 3000 or higher, 4000 or higher, 5000 or higher, 6000 or higher, 7000 or higher, 8000 or higher, 9000 or higher, 10000 or higher, 11000 or higher, 12000 or higher, 13000 or higher, 14000 or higher, or 15000 or higher.

[21] The polypeptide of any one of [20] to [20-5], wherein the activating Fcγ receptor is at least one Fcγ receptor selected from the group consisting of FcγRIa, FcγRIIa, and FcγRIIIa, and the inhibitory Fcγ receptor is FcγRIIb.

[22] The polypeptide of any one of [1] to [21], wherein the polypeptide comprising the variant Fc region is an antibody.

[23] A method for producing a polypeptide comprising a variant Fc region, which comprises introducing amino acid alterations into a parent Fc region, wherein the parent Fc region is composed of two polypeptide chains, and amino acid alterations are introduced into the following positions:

(i) positions 234, 235, 236, 239, 268, 270, and 298 according to EU numbering in the first polypeptide of the parent Fc region; and
(ii) positions 270, 298, 326, and 334 according to EU numbering in the second polypeptide of the parent Fc region.

[24] A method for producing a polypeptide comprising a variant Fc region, which comprises introducing amino acid alterations into a parent Fc region, wherein the parent Fc region is composed of two polypeptide chains, and wherein amino acid alterations are introduced into the following positions:

(i) positions 234, 235, 236, 239, 268, 270, 298, and 326 according to EU numbering in the first polypeptide of the parent Fc region; and
(ii) positions 236, 270, 298, 326, and 334 according to EU numbering in the second polypeptide of the parent Fc region.

[25] A method for producing a polypeptide comprising a variant Fc region, which comprises introducing amino acid alterations into a parent Fc region, wherein the parent Fc region is composed of two polypeptide chains, and wherein amino acid alterations are introduced into the following positions:

(i) positions 234, 235, 236, 239, 268, 270, 298, 330, and 332 according to EU numbering in the first polypeptide of the parent Fc region; and
(ii) positions 236, 270, 298, 326, 330, 332, and 334 according to EU numbering in the second polypeptide of the parent Fc region.

[26] An isolated nucleic acid encoding the polypeptide of any one of [1] to [22].

[27] A host cell comprising the nucleic acid of [26].

[28] A method for producing a polypeptide, which comprises culturing the host cell of [27] such that the polypeptide is produced.

[29] The polypeptide of any one of [1] to [22], which is for use in treatment of a tumor.

[30] The polypeptide of any one of [1] to [22], which is for use in damaging a cell.

[31] The polypeptide of [30], wherein damaging a cell is by ADCC activity, CDC activity, or ADCP activity.

[32] A pharmaceutical composition comprising the polypeptide of any one of [1] to [22] and a pharmaceutically acceptable carrier.

[33] The pharmaceutical composition of [32], which is a pharmaceutical composition for treating a tumor.

[34] The pharmaceutical composition of [32], which is a pharmaceutical composition for damaging a cell.

[35] The pharmaceutical composition of [34], wherein damaging a cell is by ADCC activity, CDC activity, or ADCP activity.

[36] A method for treating a tumor, which comprises administering the polypeptide of any one of [1] to [22] or the pharmaceutical composition of [32].

[37] A method for damaging a cell, which comprises administering the polypeptide of any one of [1] to [22] or the pharmaceutical composition of [32].

[38] The method of [37], wherein damaging a cell is by ADCC activity, CDC activity, or ADCP activity.

[39] Use of the polypeptide of any one of [1] to [22] in manufacture of an agent for treating a tumor.

[40] Use of the polypeptide of any one of [1] to [22] in manufacture of an agent for damaging a cell.

[41] The use of [40], wherein damaging a cell is by ADCC activity, CDC activity, or ADCP activity.

[42] A method for modifying a function of a polypeptide comprising a Fc region, which comprises introducing amino acid alterations into a parent Fc region, wherein the parent Fc region is composed of two polypeptide chains, and amino acid alterations are introduced into the following positions:

(i) positions 234, 235, 236, 239, 268, 270, and 298 according to EU numbering in the first polypeptide of the parent Fc region; and

(ii) positions 270, 298, 326, and 334 according to EU numbering in the second polypeptide of the parent Fc region.

[43] A method for modifying a function of a polypeptide comprising a Fc region, which comprises introducing amino acid alterations into a parent Fc region, wherein the parent Fc region is composed of two polypeptide chains, and amino acid alterations are introduced into the following positions:

(i) positions 234, 235, 236, 239, 268, 270, 298, and 326 according to EU numbering in the first polypeptide of the parent Fc region; and

(ii) positions 236, 270, 298, 326, and 334 according to EU numbering in the second polypeptide of the parent Fc region.

[44] A method for modifying a function of a polypeptide comprising a Fc region, which comprises introducing amino acid alterations into a parent Fc region, wherein the parent Fc region is composed of two polypeptide chains, and amino acid alterations are introduced into the following positions:

(i) positions 234, 235, 236, 239, 268, 270, 298, 330, and 332 according to EU numbering in the first polypeptide of the parent Fc region; and

(ii) positions 236, 270, 298, 326, 330, 332, and 334 according to EU numbering in the second polypeptide of the parent Fc region.

[45] The method of any one of [42] to [44], wherein the modification of the function is enhancement of binding activity to FcγRIIa and FcγRIIIa.

[46] The method of any one of [42] to [44], wherein the modification of the function is improvement of selectivity between an activating Fcγ receptor and an inhibitory Fcγ receptor.

[47] The method of any one of [42] to [44], wherein the modification of the function is selective enhancement of binding activity to an activating Fcγ receptor as compared to binding activity to an inhibitory Fcγ receptor.

[48] The method of any one of [42] to [44], wherein the modification of the function is an increase in a ratio of binding activity to an activating Fcγ receptor to binding activity to an inhibitory Fcγ receptor (A/I ratio).

[49] The method of [48], wherein the ratio (A/I ratio) is increased 1.1 times or more, 1.2 times or more, 1.3 times or more, 1.4 times or more, 1.5 times or more, 1.6 times or more, 1.7 times or more, 1.8 times or more, 1.9 times or more, 2 times or more, 3 times or more, 4 times or more, 5 times or more, 6 times or more, 7 times or more, 8 times or more, 9 times or more, 10 times or more, 20 times or more, 30 times or more, 40 times or more, 50 times or more, 60 times or more, 70 times or more, 80 times or more, 90 times or more, 100 times or more, 200 times or more, 300 times or more, 400 times or more, 500 times or more, 600 times or more, 700 times or more, 800 times or more, 900 times or more, 1000 times or more, 2000 times or more, 3000 times or more, 4000 times or more, 5000 times or more, 6000 times or more, 7000 times or more, 8000 times or more, 9000 times or more, or 10000 times or more compared to that in a polypeptide comprising the parent Fc region.

[50] The method of any one of [42] to [49], wherein the activating Fcγ receptor is at least one Fcγ receptor selected from the group consisting of FcγRIa, FcγRIIa, and FcγRIIIa, and the inhibitory Fcγ receptor is FcγRIIb.

[51] The method of any one of [42] to [44], wherein the modification of the function is enhancement of ADCC activity, CDC activity, or ADCP activity.

[Brief Description of Drawings]

Fig. 1 shows the result of an ADCC reporter gene assay where Hepa1-6/hEREG cells were used as target cells and Jurkat cells expressing hFcγRIIIaV were used as effector cells. Each point indicates the mean fold induction value, n=2.

Fig. 2 shows the result of an ADCC reporter gene assay where Hepa1-6/hEREG cells were used as target cells and Jurkat cells expressing hFcγRIIaH were used as effector cells. Each point indicates the mean fold induction value, n=3.

Fig. 3 shows the antitumor effect of EGL-G1d, EGL-afucosyl, and EGL-ART6 in a human FcγR transgenic mouse model into which the Hepa1-6/hEREG cell line was transplanted. The antibody was administered at 10 mg/kg via the tail vein. Each point indicates the mean tumor volume for one group, n=5.

Fig. 4 shows the hC1q-binding activity of each antibody having a modified Fc. Each point indicates the mean ELISA color development value, n=2.

Fig. 5 also shows the hC1q-binding activity of each antibody having a modified Fc. Each point indicates the mean ELISA color development value, n=2.

[Description of Embodiments]

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3rd edition (2001) Cold Spring Harbor Laboratory Press, Cold Spring Harbor, N.Y.; Current Protocols in Molecular Biology (F.M. Ausubel, et al., eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J. P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane, Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

I. DEFINITIONS

Unless defined otherwise, technical and scientific terms used herein have the same meaning as commonly understood by one skilled in the art to which the present invention belongs. Singleton et al., Dictionary of Microbiology and Molecular Biology 2nd ed., J. Wiley & Sons (New York, N.Y. 1994), and March, Advanced Organic Chemistry Reactions, Mechanisms and Structure 4th ed., John Wiley & Sons (New York, N.Y. 1992), provide one skilled in the art with a general guide to many of the terms used in the present application. All references cited herein, including patent applications and publications, are incorporated herein by reference in their entirety.

For purposes of interpreting the present specification, the following definitions apply and whenever appropriate, terms used in the singular also include the plural and vice versa. It is to be understood that the terminology used herein is for the purpose of describing particular embodiments only, and is not intended to be limiting. In the event that any definition set forth below conflicts with any document incorporated herein by reference, the definition set forth below shall control.

Herein, "first polypeptide" and "second polypeptide" mean polypeptides constituting an antibody Fc region. The terms "first polypeptide" and "second polypeptide" mean that their sequences are different from each other, and preferably at least the CH2 domain sequences are different. Further, the CH3 domain sequences may also be different. The polypeptides may be, for example, polypeptides that constitute the Fc region of a naturally-occurring (native) IgG, or polypeptides produced by altering the polypeptides constituting the Fc region of a naturally-occurring (native) IgG.

"Native IgGs" refers to polypeptides that belong to a class of antibodies practically encoded by immunoglobulin gamma genes and comprise an amino acid sequence identical to those of IgGs found in nature. For example, a native human IgG refers to a native human IgG1, native human IgG2, native human IgG3, native human IgG4, or such. Native IgGs also include mutants spontaneously produced from them.

"Polypeptides" of the present invention generally refers to peptides or proteins approximately ten amino acids or more in length. Furthermore, they are generally polypeptides derived from organisms, but are not particularly limited, and for example, they may be polypeptides comprising an artificially designed sequence. Furthermore, they may be any of naturally-occurring polypeptides, synthetic polypeptides, recombinant polypeptides, or such. Protein molecules of the present invention refer to molecules comprising the polypeptide.

Preferred examples of the polypeptides of the present invention include antibodies. More preferred examples include native IgGs and antibodies resulting from modification introduced into native IgGs. Examples of the native IgGs include, in particular, native human IgGs. "Native IgGs" refers to polypeptides belonging to a class of antibodies practically encoded by immunoglobulin gamma genes and comprising an amino acid sequence identical to those of IgGs found in nature. For example, a native human IgG means a native human IgG1, native human IgG2, native human IgG3, native human IgG4, or such. Native IgGs also include mutants spontaneously produced from them.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy chain domain or a heavy chain variable domain, followed by three constant heavy chain domains (CH1, CH2, and CH3). Similarly, from N- to C-terminus, each light chain has a variable region (VL), also called a variable light chain domain or a light chain variable domain, followed by a constant light chain (CL) domain. The light chain of an antibody may be assigned to one of two types, called kappa ($\kappa$) and lambda ($\lambda$), based on the amino acid sequence of its constant domain.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called $\alpha$, $\delta$, $\varepsilon$, $\gamma$, and $\mu$, respectively.

The term "variable region" or "variable domain" refers to the domain of an antibody heavy and/or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs) (See, *e.g.,* Kindt et al., Kuby Immunology, 6th ed., W.H. Freeman and Co., page 91 (2007)). A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, *e.g.,* Portolano et al., J. Immunol. 150: 880-887 (1993); Clarkson et al., Nature 352: 624-628 (1991).

The terms "full length antibody," "intact antibody," and "whole antibody" are used herein interchangeably to refer to an antibody having a structure substantially similar to a native antibody structure or having heavy chains that contain an Fc region as defined herein.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) or glycine-lysine (Gly446-Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

The term "polypeptide comprising an Fc region" is not particularly limited as long as it is a polypeptide that comprises an Fc region. For example, it is an antibody that comprises an Fc region. The C-terminal lysine (residue 447 according to the EU numbering system) or C-terminal glycine-lysine (residues 446-447) of the Fc region may be removed, for example, during purification of the polypeptide (e.g. antibody) or by recombinant engineering of the nucleic acid encoding the polypeptide. Accordingly, a composition comprising a polypeptide having an Fc region according to this invention can comprise a polypeptide comprising an Fc region with G446-K447, a polypeptide comprising an Fc region with G446 and without K447, a polypeptide comprising an Fc region with all G446-K447 removed, or a mixture of three types of polypeptides described above.

A "native sequence Fc region" comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

A "variant Fc region" comprises an amino acid sequence which differs from that of a native sequence Fc region by virtue of at least one amino acid modification (alteration), preferably one or more amino acid substitution(s). Preferably, the variant Fc region has at least one amino acid substitution compared to a native sequence Fc region or to the parent Fc region, *e.g.*, from about 1 to about 30 amino acid substitutions, preferably from about 1 to about 20 amino acid substitutions, more preferably from about 1 to about 10 amino acid substitutions, and most preferably from about 1 to about 5 amino acid substitutions in a native sequence Fc region or in the parent Fc region. The variant Fc region herein preferably possesses at least about 80% homology with a native sequence Fc region or with a parent Fc region, preferably at least about 85% homology therewith, more preferably at least about 90% homology therewith, and most preferably at least about 95% homology therewith.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR) software, or GENETYX (registered trademark) (Genetyx Co., Ltd.). Those skilled in the art can determine appropriate parameters for aligning sequences, including any algorithms needed to achieve maximal alignment over the full length of the sequences being compared.

The term "Fc receptor" or "FcR" refers to a receptor that binds to the Fc region of an antibody. In some embodiments, an FcR is a native human FcR. In some embodiments, an FcR is one which binds an IgG antibody (a gamma receptor) and includes receptors of the FcγRI, FcγRII, and FcγRIII subclasses, including allelic variants and alternatively spliced forms of those receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (see, *e.g.,* Daeron, Annu. Rev. Immunol. 15: 203-234 (1997)). FcRs are reviewed, for example, in Ravetch and Kinet, Annu. Rev. Immunol 9: 457-492 (1991); Capel et al., Immunomethods 4: 25-34 (1994); and de Haas et al., J. Lab. Clin. Med. 126: 330-341 (1995). Other FcRs, including those to be identified in the future, are encompassed by the term "FcR" herein.

The term "Fc receptor" or "FcR" also includes the neonatal receptor, FcRn, which is responsible for the transfer of maternal IgGs to the fetus (Guyer et al., J. Immunol. 117: 587 (1976); and Kim et al., J. Immunol. 24: 249 (1994)) and regulation of homeostasis of immunoglobulins. Methods of measuring binding to FcRn are known (see, *e.g.*, Ghetie and Ward., Immunol. Today 18(12): 592-598 (1997); Ghetie et al., Nature Biotechnology, 15(7): 637-640 (1997); Hinton et al., J. Biol. Chem. 279(8): 6213-6216 (2004); WO 2004/92219 (Hinton et al.)).

"Effector cells" refer to leukocytes that express one or more FcRs and perform effector functions. In certain embodiments, the cells express at least FcγRIII and perform ADCC effector function(s). Examples of leukocytes which mediate ADCC include peripheral blood mononuclear cells (PBMC), natural killer (NK) cells, monocytes, cytotoxic T cells, and neutrophils. The effector cells may be isolated from a native source, *e.g.*, from blood. In certain embodiments, the effector cells may be human effector cells.

"Effector functions" refer to those biological activities attributable to the Fc region of an antibody, which vary with the antibody isotype. Examples of antibody effector functions include: C1q binding and complement-dependent cytotoxicity (CDC); Fc receptor binding; antibody-dependent cell-mediated cytotoxicity (ADCC); antibody-dependent cell-mediated phagocytosis (ADCP); down regulation of cell surface receptors (*e.g.*, B cell receptor); and B cell activation.

A "functional Fc region" possesses an "effector function" of a native sequence Fc region. Such effector functions generally require the Fc region to be combined with a binding domain (*e.g.*, an antibody variable domain) and can be assessed using various assays as disclosed, for example, in definitions herein.

"Antibody-dependent cell-mediated cytotoxicity" or "ADCC" refers to a form of cytotoxicity in which secreted immunoglobulin bound onto Fc receptors (FcRs) present on certain cytotoxic cells (*e.g.*, NK cells, neutrophils, and macrophages) enable these cytotoxic effector cells to bind specifically to an antigen-bearing target cell and subsequently kill the target cell with cytotoxins. The primary cells for mediating ADCC, NK cells, express FcγRIII only, whereas monocytes express FcγRI, FcγRII, and FcγRIII. FcR expression on hematopoietic cells is summarized in Table 3 on page 464 of Ravetch and Kinet, Annu. Rev. Immunol 9: 457-92 (1991). To assess ADCC activity of a molecule of interest, an *in vitro* ADCC assay, such as that described in US Patent No. 5,500,362 or 5,821,337 or U.S. Patent No. 6,737,056 (Presta), may be performed. Useful effector cells for such assays include PBMC and NK cells. Alternatively, or additionally, ADCC activity of the molecule of interest may be assessed *in vivo*, *e.g.*, in an animal model such as that disclosed in Clynes et al. PNAS (USA) 95: 652-656 (1998).

"Cytotoxic activity" includes, for example, antibody-dependent cell-mediated cytotoxicity (ADCC) activity as mentioned above, complement-dependent cytotoxicity (CDC) activity as mentioned below, and T cell-mediated cytotoxic activity. CDC activity means the cytotoxic activity by the complement system. On the other hand, ADCC activity means the activity in which an antibody binds to an antigen present on the cell surface of a target cell and an effector cell further binds to the antibody, and thereby the effector cell damages the target cell. Whether an antibody of interest has ADCC activity and whether an antibody of interest has CDC activity can be measured by known methods (for example, Current Protocols in Immunology, Chapter 7, Immunologic studies in humans, edited by Coligan et al. (1993)).

The term "complement-dependent cytotoxicity" or "CDC" means a mechanism for inducing cell death in which the Fc effector domain of an antibody bound to a target activates a series of enzymatic reactions, resulting in the formation of holes in the membrane of the target cell. Typically, the antigen-antibody complex formed on the target cell binds to and activates the complement component C1q, which in turn activates the complement cascade and leads to the target cell death. Moreover, complement activation may also result in the deposition of complement components on the surface of the target cell, which leads to binding to complement receptors (e.g., CR3) on leukocytes and thereby promotes ADCC.

The term "antibody-dependent cellular phagocytosis" or "ADCP" means the process in which either the whole or part of a cell covered by an antibody is incorporated into phagocytic immune cells (*e.g.*, macrophages, neutrophils, and dendritic cells) that bind to an immunoglobulin Fc region.

An "isolated" polypeptide is one which has been separated from a component of its natural environment. In some embodiments, a polypeptide is purified to greater than 95% or 99% purity as determined by, for example, electrophoretic (*e.g.*, SDS-PAGE, isoelectric focusing (IEF), capillary electrophoresis) or chromatographic (*e.g.*, ion exchange or reverse phase HPLC). For review of methods for assessment of polypeptide purity, see, *e.g.,* Flatman et al., J. Chromatogr. B 848: 79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding a polypeptide" refers to one or more nucleic acid molecules encoding the polypeptide (e.g. antibody Fc region or antibody heavy and light chains or fragments thereof), including such nucleic acid molecule(s) in a single vector or separate vectors, and such nucleic acid molecule(s) present at one or more locations in a host cell.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

The terms "pharmaceutical formulation" and "pharmaceutical composition" are interchangeably used, and refer to a preparation which is in such form as to permit the biological activity of an active ingredient contained therein to be effective, and which contains no additional components which are unacceptably toxic to a subject to which it would be administered.

An "individual" or "subject" is a mammal. Mammals include, but are not limited to, domesticated animals (*e.g.*, cows, sheep, cats, dogs, and horses), primates (*e.g.*, humans and non-human primates such as monkeys), rabbits, and rodents (*e.g.*, mice and rats). In certain embodiments, the individual or subject is a human.

A "pharmaceutically acceptable carrier" refers to an ingredient in a pharmaceutical formulation and a pharmaceutical composition, other than an active ingredient, which is nontoxic to a subject. A pharmaceutically acceptable carrier includes, but is not limited to, a buffer, excipient, stabilizer, or preservative.

An "effective amount" of an agent, *e.g.*, a pharmaceutical formulation, refers to an amount effective, at dosages and for periods of time necessary, to achieve the desired therapeutic or prophylactic result.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, polypeptides comprising a variant Fc region of the present invention are used to delay development of a disease or to slow the progression of a disease.

The term "tumor" refers to all neoplastic cell growth and proliferation, whether malignant or benign, and all precancerous and cancerous cells and tissues. The terms "cancer," "cancerous," "cell proliferative disorder," "proliferative disorder" and "tumor" are not mutually exclusive as referred to herein.

The term "tumor tissue" means a tissue comprising at least one tumor cell. Tumor tissue is usually composed of a population of tumor cells that form the main entity of the tumor (parenchyma) and connective tissues and blood vessels that exist between these cells and support the tumor ("stroma"). The distinction between the two is clear in some cases, while in others they are intermingled. Tumor tissues may be infiltrated by immune cells and the like. On the other hand, a "non-tumor tissue" means a tissue other than a tumor tissue(s) in a living organism. Healthy/normal tissues that are not in a diseased state are typical examples of non-tumor tissues.

< Polypeptides comprising a variant Fc region >

In one aspect, the present invention provides isolated polypeptides comprising a variant Fc region. In some aspects, the polypeptides are antibodies. In some aspects, the polypeptides are Fc fusion proteins. In certain embodiments, the variant Fc regions comprise at least one amino acid residue alteration (*e.g.*, substitution) compared to the corresponding sequence in the Fc region of a native sequence or a reference variant sequence (herein may be collectively referred to as the "parent" Fc region). The Fc region of a native sequence is usually composed as a homodimer consisting of two identical polypeptide chains. The amino acid alterations in the variant Fc regions in the present invention may be introduced into either one of the two polypeptide chains of the parent Fc region, or into both of the two polypeptide chains.

In some aspects, the present invention provides variant Fc regions whose function has been modified compared to the parent Fc region. In certain aspects, the variant Fc regions in the present invention have enhanced binding activity to Fcγ receptor compared to the parent Fc region. In certain embodiments, the variant Fc regions in the present invention have enhanced binding activity to at least one Fcγ receptor selected from the group consisting of FcγRIa, FcγRIIa, FcγRIIb, and FcγRIIIa, compared to the parent Fc region. In some embodiments, the variant Fc regions in the present invention have enhanced binding activity to FcγRIIa. In some embodiments, the variant Fc regions in the present invention have enhanced binding activity to FcγRIIIa. In further embodiments, the variant Fc regions in the present invention have enhanced binding activity to FcγRIIa and FcγRIIIa. In another aspect, the variant Fc regions in the present invention have enhanced ADCC activity, CDC activity, or ADCP activity as compared to the parent Fc region.

The terms "binding activity" and "binding ability" are interchangeably used herein, and refer to the strength of the sum total of noncovalent interactions between one or more binding sites (*e.g.* a variable region or Fc region) of a molecule (*e.g.*, an antibody or other polypeptide) and its binding partner (*e.g.*, an antigen or Fcγ receptor). Herein, "binding activity" is not strictly limited to a 1:1 interaction between members of a binding pair (*e.g.*, antibody and antigen, or Fc region and Fcγ receptor). For example, when members of a binding pair reflect a monovalent 1:1 interaction, the binding activity refers to the intrinsic binding affinity ("affinity"). When a member of a binding pair is capable of both monovalent and multivalent binding, the binding activity is the sum of each binding strength. The binding activity of a molecule X to its partner Y can generally be represented by the dissociation constant (KD) or "binding amount of analyte per unit amount of ligand". Binding activity can be measured by common methods known in the art, including those described herein. Specific illustrative and exemplary embodiments for measuring binding activity are described in the following.

In certain embodiments, the binding activity of the parent Fc region and the variant Fc regions can be represented by a KD (dissociation constant) value. In one embodiment, the value of the ratio of [KD value of the parent Fc region for FcγRIIa] / [KD value of a variant Fc region for FcγRIIa] is, for example, 1.5 or more, 2 or more, 3 or more, 4 or more, 5 or more, 6 or more, 7 or more, 8 or more, 9 or more, 10 or more, 15 or more, 20 or more, 25 or more, 30 or more, 40 or more, or 50 or more. In further embodiments, FcγRIIa may be FcγRIIa R or FcγRIIa H, or may also be both. Therefore, the KD value of an Fc region for FcγRIIa may be a KD value of the Fc region for FcγRIIa R, a KD value of the Fc region for FcγRIIa H, or the sum or average of both. In one embodiment, the value of the ratio of [binding activity of the parent Fc region to FcγRIIIa] / [binding activity of a variant Fc region to FcγRIIIa] is, for example, 2 or more, 3 or more, 5 or more, 10 or more, 20 or more, 30 or more, 50 or more, 100 or more, 200 or more, 300 or more, 500 or more, $1 \times 10^3$ or more, $2 \times 10^3$ or more, $3 \times 10^3$ or more, or $5 \times 10^3$ or more. In further embodiments, FcγRIIIa may be FcγRIIIa F or FcγRIIIa V, or may also be both. Therefore, the KD value of an Fc region for FcγRIIIa may be a KD value of the Fc region for FcγRIIIa F, a KD value of the Fc region for FcγRIIa V, or the sum or average of both.

In one embodiment, the KD values of the variant Fc regions for FcγRIIa are, for example, $1.0 \times 10^{-6}$ M or less, $5.0 \times 10^{-7}$ M or less, $3.0 \times 10^{-7}$ M or less, $2.0 \times 10^{-7}$ M or less, $1.0 \times 10^{-7}$ M or less, $5.0 \times 10^{-8}$ M or less, $3.0 \times 10^{-8}$ M or less, $2.0 \times 10^{-8}$ M or less, $1.0 \times 10^{-8}$ M or less, $5.0 \times 10^{-9}$ M or less, $3.0 \times 10^{-9}$ M or less, $2.0 \times 10^{-9}$ M or less, or $1.0 \times 10^{-9}$ M or less. In further embodiments, FcγRIIa may be FcγRIIa R or FcγRIIa H, or may also be both. In one embodiment, the KD values of the variant Fc regions for FcγRIIIa are, for example, $1.0 \times 10^{-6}$ M or less, $5.0 \times 10^{-7}$ M or less, $3.0 \times 10^{-7}$ M or less, $2.0 \times 10^{-7}$ M or less, $1.0 \times 10^{-7}$ M or less, $5.0 \times 10^{-8}$ M or less, $3.0 \times 10^{-8}$ M or less, 2.0 × 10⁻⁸ M or less, 1.0 × 10⁻⁸ M or less, 5.0 × 10⁻⁹ M or less, 3.0 × 10⁻⁹ M or less, 2.0 × 10⁻⁹ M or less, 1.0 × 10⁻⁹ M or less, 5.0 × 10⁻¹⁰ M or less, 3.0 × 10⁻¹⁰ M or less, 2.0 × 10⁻¹⁰ M or less, or 1.0 × 10⁻¹⁰ M or less. In further embodiments, FcγRIIIa may be FcγRIIIa For FcγRIIIa V, or may also be both.

In another embodiment, the binding activity of the parent and variant Fc regions may be represented by a kd (dissociation rate constant) value instead of a KD value.

In another embodiment, the binding activity of the parent and variant Fc regions may be represented by the binding amount to an Fcγ receptor per unit amount of the Fc region. For example, in a surface plasmon resonance assay, the binding amount of an Fc region immobilized onto a sensor chip and the binding amount of an Fcγ receptor further bound thereto are each measured as a resonance unit (RU). The value obtained by dividing the binding amount of the Fcγ receptor by the binding amount of the Fc region can be defined as the binding amount to the Fcγ receptor per unit amount of the Fc region. Specific methods for measuring and calculating such binding amounts are described in Examples below. In some embodiments, the value of the ratio of [binding amount of a variant Fc region to FcγRIIa] / [binding amount of the parent Fc region to FcγRIIa] is, for example, 1.5 or more, 2 or more, 3 or more, 4 or more, 5 or more, 6 or more, 7 or more, 8 or more, 9 or more, 10 or more, 15 or more, 20 or more, 25 or more, 30 or more, or 40 or more, or 50 or more. In some embodiments, the value of the ratio of [binding amount of a variant Fc region to FcγRIIIa] / [binding amount of the parent Fc region to FcγRIIIa] is, for example, 2 or more, 3 or more, 5 or more, 10 or more, 20 or more, 30 or more, 50 or more, 100 or more, 200 or more, 300 or more, 500 or more, 1 × 10³ or more, 2 × 10³ or more, 3 × 10³ or more, or 5 × 10³ or more.

In certain embodiments, the KD values, kd values, values of binding amount and such described herein are measured or calculated by performing a surface plasmon resonance assay at 25°C or 37°C (see, for example, Example 2 herein).

In certain aspects, the variant Fc region in the present invention has improved selectivity between activating and inhibitory Fcγ receptors compared to the parent Fc region. In other words, the binding activity of the variant Fc region in the present invention to an activating Fcγ receptor is greatly enhanced than that to an inhibitory Fcγ receptor, compared to the parent Fc region. In certain embodiments, the activating Fcγ receptor is at least one Fcγ receptor selected from the group consisting of FcγRIa, FcγRIIa R, FcγRIIa H, FcγRIIIa F, and FcγRIIIa V, and the inhibitory Fcγ receptor is FcγRIIb. In some embodiments, the variant Fc regions in the present invention have improved selectivity between FcγRIIa and FcγRIIb. In some embodiments, the variant Fc region in the present invention has improved selectivity between FcγRIIIa and FcγRIIb. In further embodiments, the variant Fc regions in the present invention have improved selectivity between FcγRIIa and FcγRIIb and between FcγRIIIa and FcγRIIb.

In certain embodiments, the binding activity of the parent and variant Fc regions can be represented by a KD (dissociation constant) value. The embodiments of the binding activity to FcγRIIa and FcγRIIIa are as described above. In one embodiment, the value of the ratio of [KD value of the parent Fc region for FcγRIIb] / [KD value of a variant Fc region for FcγRIIb] is, for example, 10 or less, 5 or less, 3 or less, 2 or less, 1 or less, 0.5 or less, 0.3 or less, 0.2 or less, or 0.1 or less. In another embodiment, the binding activity of the parent and variant Fc regions may be represented by a kd (dissociation rate constant) value instead of a KD value.

In another embodiment, the binding activity of the parent and variant Fc regions may be represented by the above-mentioned binding amount to an Fcγ receptor per unit amount of the Fc region. In some embodiments, the value of the ratio of [binding amount of a variant Fc region to FcγRIIb] / [binding amount of the parent Fc region to FcγRIIb] is, for example, 10 or less, 5 or less, 3 or less, 2 or less, 1 or less, 0.5 or less, 0.3 or less, 0.2 or less, or 0.1 or less. In some embodiments, the binding amount of the variant Fc region to FcγRIIb is, for example, 0.5 or less, 0.3 or less, 0.2 or less, 0.1 or less, 0.05 or less, 0.03 or less, 0.02 or less, 0.01 or less, 0.005 or less, 0.003 or less, 0.002 or less, or 0.001 or less.

In a certain embodiment, the improvement of selectivity between an activating Fcγ receptor and an inhibitory Fcγ receptor is selective enhancement of binding activity to an activating Fcγ receptor as compared to binding activity to an inhibitory Fcγ receptor, or in other words, an increase in the ratio of binding activity to an activating Fcγ receptor to binding activity to an inhibitory Fcγ receptor (All ratio). This ratio (A/I ratio) is an index for exertion of excellent effector functions. Polypeptides with a high A/I ratio can be evaluated as having excellent effector functions. The binding activity of a parent Fc region and a variant Fc region to an Fcγ receptor can be represented by a KD value, a kd value, or the binding amount of the Fc region to the Fcγ receptor per unit amount. The All ratio can be expressed using KD values, kd values, or binding amounts as follows: [KD value for an inhibitory Fcγ receptor]/[KD value for an activating Fcγ receptor], [kd value for an inhibitory Fcγ receptor]/[kd value for an activating Fcγ receptor], or [binding amount to an activating Fcγ receptor]/[binding amount to an inhibitory Fcγ receptor].

In one embodiment, the All ratio of the variant Fc region of the present invention is increased 1.1 times or more, 1.2 times or more, 1.3 times or more, 1.4 times or more, 1.5 times or more, 1.6 times or more, 1.7 times or more, 1.8 times or more, 1.9 times or more, 2 times or more, 3 times or more, 4 times or more, 5 times or more, 6 times or more, 7 times or more, 8 times or more, 9 times or more, 10 times or more, 20 times or more, 30 times or more, 40 times or more, 50 times or more, 60 times or more, 70 times or more, 80 times or more, 90 times or more, 100 times or more, 200 times or more, 300 times or more, 400 times or more, 500 times or more, 600 times or more, 700 times or more, 800 times or more, 900 times or more, 1000 times or more, 2000 times or more, 3000 times or more, 4000 times or more, 5000 times or more, 6000 times or more, 7000 times or more, 8000 times or more, 9000 times or more, or 10000 times or more as compared to that of the parent Fc region. In one embodiment, the A/I ratio of the variant Fc region of the present invention has a value of 10 or higher, 20 or higher, 30 or higher, 40 or higher, 50 or higher, 60 or higher, 70 or higher, 80 or higher, 90 or higher, 100 or higher, 200 or higher, 300 or higher, 400 or higher, 500 or higher, 600 or higher, 700 or higher, 800 or higher, 900 or higher, 1000 or higher, 2000 or higher, 3000 or higher, 4000 or higher, 5000 or higher, 6000 or higher, 7000 or higher, 8000 or higher, 9000 or higher, 10000 or higher, 11000 or higher, 12000 or higher, 13000 or higher, 14000 or higher, or 15000 or higher. In one embodiment, the A/I ratio is a ratio of binding activity to FcyRIa to binding activity to FcyRIIb, a ratio of binding activity to FcyRIIa to binding activity to FcyRIIb, a ratio of binding activity to FcyRIIIa to binding activity to FcyRIIb, or a ratio of [the sum or average of two or three of binding activity to FcyRIa, binding activity to FcyRIIa, and binding activity to FcyRIIIa] to binding activity to FcyRIIb. In a certain embodiment, FcyRIIa is FcyRIIa R, FcyRIIa H, or both. Thus, the binding activity to FcyRIIa is binding activity to FcyRIIa R, binding activity to FcyRIIa H, or the sum or average of both. In a certain embodiment, FcyRIIIa is FcyRIIIa F, FcyRIIIa V, or both. Thus, the binding activity to FcyRIIIa is binding activity to FcyRIIIa F, binding activity to FcyRIIIa V, or the sum or average of both.

In some embodiments, the variant Fc region of the present invention comprises amino acid alterations at the following positions:

(i) positions 234, 235, 236, 239, 268, 270, and 298 according to EU numbering in the first polypeptide of the parent Fc region; and
(ii) positions 270, 298, 326, and 334 according to EU numbering in the second polypeptide of the parent Fc region.

In a certain embodiment, the variant Fc region of the present invention further comprises an amino acid alteration at position 326 according to EU numbering in the first polypeptide of the parent Fc region. In a certain embodiment, the variant Fc region of the present invention further comprises an amino acid alteration at position 236 according to EU numbering in the second polypeptide of the parent Fc region. In a certain embodiment, the variant Fc region of the present invention further comprises an amino acid alteration at position 332 according to EU numbering in the first polypeptide of the parent Fc region. In a certain embodiment, the variant Fc region of the present invention further comprises an amino acid alteration at position 330 according to EU numbering in the first polypeptide of the parent Fc region. In a certain embodiment, the variant Fc region of the present invention further comprises an amino acid alteration at position 332 according to EU numbering in the second polypeptide of the parent Fc region. In a certain embodiment, the variant Fc region of the present invention further comprises an amino acid alteration at position 330 according to EU numbering in the second polypeptide of the parent Fc region. Alternatively, the amino acid alterations described in International Publications WO2013/002362 and WO2014/104165 may also be similarly used in the present invention.

In some embodiments, the variant Fc region of the present invention comprises amino acid alterations at the following positions:

(i) positions 234, 235, 236, 239, 268, 270, 298, and 330 according to EU numbering in the first polypeptide of the parent Fc region; and
(ii) positions 270, 298, 326, 330, and 334 according to EU numbering in the second polypeptide of the parent Fc region.

In a certain embodiment, the variant Fc region of the present invention further comprises an amino acid alteration at position 236 according to EU numbering in the second polypeptide of the parent Fc region. In a certain embodiment, the variant Fc region of the present invention further comprises an amino acid alteration at position 332 according to EU numbering in the first polypeptide of the parent Fc region. In a certain embodiment, the variant Fc region of the present invention further comprises an amino acid alteration at position 332 according to EU numbering in the second polypeptide of the parent Fc region. In a certain embodiment, alternatively, the amino acid alterations described in International Publications WO2013/002362 and WO2014/104165 may also be similarly used in the present invention.

In certain aspects, the variant Fc regions in the present invention have improved stability compared to the parent Fc region. In certain embodiments, the stability is thermodynamic stability. The thermodynamic stability of a polypeptide can be judged, for example, by using the Tm value as an indicator. Tm values can be measured using techniques known to those skilled in the art, such as circular dichroism (CD), differential scanning calorimeter (DSC), and differential scanning fluorimetry (DSF). In one embodiment, in the variant Fc region in the present invention, the Tm value of the CH2 region is increased by 0.1 degrees or more, 0.2 degrees or more, 0.3 degrees or more, 0.4 degrees or more, 0.5 degrees or more, 1 degree or more, 2 degrees or more, 3 degrees or more, 4 degrees or more, 5 degrees or more, or 10 degrees or more, compared to that in the parent Fc region.

In some embodiments, the variant Fc regions in the present invention comprise at least one amino acid alteration at at least one position selected from the group consisting of positions 250 and 307 according to EU numbering in the first polypeptide and/or the second polypeptide of the parent Fc region. Alternatively, the amino acid alterations described in WO 2013/118858 can be similarly used in the present invention.

In certain aspects, the variant Fc region in the present invention is composed of two polypeptide chains with different sequences from each other. In a further aspect, in the variant Fc region in the present invention, heterodimerization between a first polypeptide and a second polypeptide has been promoted. When a heterodimeric protein is produced using a recombination method, it is preferable that peptide chains different from each other preferentially associate to form a heterodimer, rather than that identical polypeptide chains associate to form a homodimer. Whether heterodimerization in a variant Fc region is promoted or not can be judged, for example, by separating homodimers and heterodimers from the produced variant Fc regions by a technique such as chromatography, and by determining the ratio of each component.

In some embodiments, the variant Fc regions in the present invention comprise at least one amino acid alteration at at least one position selected from the group consisting of positions 349, 356, 366, 368, 407, and 439 according to EU numbering in the first polypeptide and/or the second polypeptide of the parent Fc region. Alternatively, the amino acid alterations described in WO 2006/106905 and WO 1996/027011 can be similarly used in the present invention.

In certain aspects, the variant Fc region in the present invention has enhanced binding activity to FcRn under acidic pH. In some embodiments, the acidic pH means pH 4.0 to 6.5. In further embodiments, the acidic pH is at least one selected from the group consisting of pH 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, and 6.5. In certain embodiments, the acidic pH is pH 5.8.

In some embodiments, the variant Fc regions in the present invention comprise at least one amino acid alteration at at least one position selected from the group consisting of positions 428, 434, 436, 438, and 440 according to EU numbering in the first polypeptide and/or the second polypeptide of the parent Fc region. Alternatively, the amino acid alterations described in WO 2016/125495 can be similarly used in the present invention.

In one aspect, the variant Fc region in the present invention comprises at least one amino acid alteration at at least one position selected from the group consisting of positions 234, 235, 236, 239, 250, 268, 270, 298, 307, 326, 330, 332, 334, 349, 356, 366, 368, 407, 428, 434, 436, 438, 439, and 440 according to EU numbering.

In one embodiment, the variant Fc region of the present invention comprises amino acid alterations at positions 234, 235, 236, 239, 268, 270, 298, 326, and 334 according to EU numbering. In a certain embodiment, the variant Fc region of the present invention comprises amino acid alterations at (i) positions 234, 235, 236, 239, 268, 270, and 298 according to EU numbering in the first polypeptide of the parent Fc region, and (ii) positions 270, 298, 326, and 334 according to EU numbering in the second polypeptide of the parent Fc region. In another certain embodiment, the variant Fc region of the present invention comprises amino acid alterations at (i) positions 234, 235, 236, 239, 268, 270, 298, and 326 according to EU numbering in the first polypeptide of the parent Fc region, and (ii) positions 236, 270, 298, 326, and 334 according to EU numbering in the second polypeptide of the parent Fc region.

In one embodiment, the variant Fc region of the present invention comprises amino acid alterations at positions 234, 235, 236, 239, 268, 270, 298, 326, 330, and 334 according to EU numbering. In a certain embodiment, the variant Fc region of the present invention comprises amino acid alterations at (i) positions 234, 235, 236, 239, 268, 270, 298, and 330 according to EU numbering in the first polypeptide of the parent Fc region, and (ii) positions 270, 298, 326, 330, and 334 according to EU numbering in the second polypeptide of the parent Fc region.

In one embodiment, the variant Fc region of the present invention comprises amino acid alterations at positions 234, 235, 236, 239, 250, 268, 270, 298, 307, 326, and 334 according to EU numbering. In a certain embodiment, the variant Fc region of the present invention comprises amino acid alterations at (i) positions 234, 235, 236, 239, 250, 268, 270, 298, and 307 according to EU numbering in the first polypeptide of the parent Fc region, and (ii) positions 250, 270, 298, 307, 326, and 334 according to EU numbering in the second polypeptide of the parent Fc region. In another certain embodiment, the variant Fc region of the present invention comprises amino acid alterations at (i) positions 234, 235, 236, 239, 250, 268, 270, 298, 307, and 326 according to EU numbering in the first polypeptide of the parent Fc region, and (ii) positions 236, 250, 270, 298, 307, 326, and 334 according to EU numbering in the second polypeptide of the parent Fc region.

In one embodiment, the variant Fc region of the present invention comprises amino acid alterations at positions 234, 235, 236, 239, 268, 270, 298, 326, 330, 332, and 334 according to EU numbering. In a certain embodiment, the variant Fc region of the present invention comprises amino acid alterations at (i) positions 234, 235, 236, 239, 268, 270, 298, 330, and 332 according to EU numbering in the first polypeptide of the parent Fc region, and (ii) positions 236, 270, 298, 326, 330, 332, and 334 according to EU numbering in the second polypeptide of the parent Fc region.

In one embodiment, the variant Fc region of the present invention comprises amino acid alterations at positions 234, 235, 236, 239, 250, 268, 270, 298, 307, 326, 330, 332, and 334 according to EU numbering. In a certain embodiment, the variant Fc region of the present invention comprises amino acid alterations at (i) positions 234, 235, 236, 239, 250, 268, 270, 298, 307, 330, and 332 according to EU numbering in the first polypeptide of the parent Fc region, and (ii) positions 236, 250, 270, 298, 307, 326, 330, 332, and 334 according to EU numbering in the second polypeptide of the parent Fc region. In another certain embodiment, the variant Fc region of the present invention comprises amino acid alterations at (i) positions 234, 235, 236, 239, 250, 268, 270, 298, 307, and 326 according to EU numbering in the first polypeptide of the parent Fc region, and (ii) positions 236, 250, 270, 298, 307, 326, 330, 332, and 334 according to EU numbering in the second polypeptide of the parent Fc region.

In one embodiment, the variant Fc region of the present invention comprises amino acid alterations at positions 234, 235, 236, 239, 250, 268, 270, 298, 307, 326, 332, and 334 according to EU numbering. In a certain embodiment, the variant Fc region of the present invention comprises amino acid alterations at (i) positions 234, 235, 236, 239, 250, 268, 270, 298, 307, 326, and 332 according to EU numbering in the first polypeptide of the parent Fc region, and (ii) positions 236, 250, 270, 298, 307, 326, 332, and 334 according to EU numbering in the second polypeptide of the parent Fc region.

In one embodiment, the variant Fc region of the present invention comprises amino acid alterations at positions 234, 235, 236, 239, 250, 268, 270, 298, 307, 326, 332, and 334 according to EU numbering. In a certain embodiment, the variant Fc region of the present invention comprises amino acid alterations at (i) positions 234, 235, 236, 239, 250, 268, 270, 298, 307, and 332 according to EU numbering in the first polypeptide of the parent Fc region, and (ii) positions 250, 270, 298, 307, 326, 332, and 334 according to EU numbering in the second polypeptide of the parent Fc region.

In one embodiment, the variant Fc region of the present invention comprises amino acid alterations at positions 234, 235, 236, 239, 250, 268, 270, 298, 307, 326, 330, and 334 according to EU numbering. In a certain embodiment, the variant Fc region of the present invention comprises amino acid alterations at (i) positions 234, 235, 236, 239, 250, 268, 270, 298, 307, and 330 according to EU numbering in the first polypeptide of the parent Fc region, and (ii) positions 250, 270, 298, 307, 326, 330, and 334 according to EU numbering in the second polypeptide of the parent Fc region.

In further embodiments, the variant Fc regions in the present invention comprise at least one amino acid alteration selected from the group consisting of (i) Tyr or Phe at position 234, Gln or Tyr at position 235, Trp at position 236, Met at position 239, Val at position 250, Asp at position 268, Glu at position 270, Ala at position 298, Pro at position 307, Asp at position 326, Met at position 330, Glu at position 332, according to EU numbering, in the first polypeptide of the parent Fc region; and (ii) Ala at position 236, Val at position 250, Glu at position 270, Ala at position 298, Pro at position 307, Asp at position 326, Met or Lys at position 330, Asp or Glu at position 332, Glu at position 334, according to EU numbering, in the second polypeptide of the parent Fc region.

In a further embodiment, the variant Fc region of the present invention further comprises any of the amino acid alterations of (a) to (f) below:

(a) Lys at position 356 according to EU numbering in the first polypeptide of the parent Fc region, and Glu at position 439 according to EU numbering in the second polypeptide of the parent Fc region;
(b) Glu at position 439 according to EU numbering in the first polypeptide of the parent Fc region, and Lys at position 356 according to EU numbering in the second polypeptide of the parent Fc region;
(c) Trp at position 366 according to EU numbering in the first polypeptide of the parent Fc region, and Ser at position 366, Ala at position 368, and Val at position 407 according to EU numbering in the second polypeptide of the parent Fc region;
(d) Ser at position 366, Ala at position 368, and Val at position 407 according to EU numbering in the first polypeptide of the parent Fc region, and Trp at position 366 according to EU numbering in the second polypeptide of the parent Fc region;
(e) Cys at position 349 and Trp at position 366 according to EU numbering in the first polypeptide of the parent Fc region, and Cys at position 356, Ser at position 366, Ala at position 368, and Val at position 407 according to EU numbering in the second polypeptide of the parent Fc region;
(f) Cys at position 356, Ser at position 366, Ala at position 368, and Val at position 407 according to EU numbering in the first polypeptide of the parent Fc region, and Cys at position 349 and Trp at position 366 according to EU numbering in the second polypeptide of the parent Fc region.

In further aspects, the variant Fc regions in the present invention further comprise any of the amino acid alterations (a) to (d) below in the first polypeptide and/or second polypeptide of the parent Fc region:

(a) Ala at position 434 according to EU numbering;
(b) Ala at position 434, Thr at position 436, Arg at position 438, and Glu at position 440, according to EU numbering;
(c) Leu at position 428, Ala at position 434, Thr at position 436, Arg at position 438, and Glu at position 440, according to EU numbering; and
(d) Leu at position 428, Ala at position 434, Arg at position 438, and Glu at position 440, according to EU numbering.

In a certain embodiment, a polypeptide comprising the variant Fc region of the present invention is an antibody heavy chain constant region.

In further embodiments, the present invention provides polypeptides comprising the amino acid sequence of any one of SEQ ID NOs: 7-22 and 35-50.

In further embodiments, the present invention provides a heavy chain constant region comprising the polypeptide chain of SEQ ID NO: 35 and the polypeptide chain of SEQ ID NO: 36, a heavy chain constant region comprising the polypeptide chain of SEQ ID NO: 37 and the polypeptide chain of SEQ ID NO: 38, a heavy chain constant region comprising the polypeptide chain of SEQ ID NO: 39 and the polypeptide chain of SEQ ID NO: 40, a heavy chain constant region comprising the polypeptide chain of SEQ ID NO: 41 and the polypeptide chain of SEQ ID NO: 42, a heavy chain constant region comprising the polypeptide chain of SEQ ID NO: 43 and the polypeptide chain of SEQ ID NO: 44, a heavy chain constant region comprising the polypeptide chain of SEQ ID NO: 45 and the polypeptide chain of SEQ ID NO: 46, a heavy chain constant region comprising the polypeptide chain of SEQ ID NO: 47 and the polypeptide chain of SEQ ID NO: 48, and a heavy chain constant region comprising the polypeptide chain of SEQ ID NO: 49 and the polypeptide chain of SEQ ID NO: 50.

"Fcγ receptors" (herein, referred to as Fcγ receptors, FcγR or FcgR) refers to receptors that may bind to the Fc region of IgG1, IgG2, IgG3, and IgG4 monoclonal antibodies, and practically means any member of the family of proteins encoded by the Fcγ receptor genes. In humans, this family includes FcγRI (CD64) including isoforms FcγRIa, FcγRIb, and FcγRIc; FcγRII (CD32) including isoforms FcγRIIa (including allotypes H131 (type H) and R131 (type R)), FcγRIIb (including FcγRIIb-1 and FcγRIIb-2), and FcγRIIc; and FcγRIII (CD16) including isoforms FcγRIIIa (including allotypes V158 and F158), and FcγRIIIb (including allotypes FcγRIIIb-NA1 and FcγRIIIb-NA2), and any human FcγRs, FcγR isoforms or allotypes yet to be discovered, but is not limited thereto. FcγRIIb1 and FcγRIIb2 have been reported as splicing variants of human FcγRIIb. In addition, a splicing variant named FcγRIIb3 has been reported (J Exp Med, 1989, 170: 1369-1385). In addition to these splicing variants, human FcγRIIb includes all splicing variants registered in NCBI, which are NP_001002273.1, NP_001002274.1, NP_001002275.1, NP_001177757.1, and NP_003992.3. Furthermore, human FcγRIIb includes every previously-reported genetic polymorphism, as well as FcγRIIb (Arthritis Rheum. 48: 3242-3252 (2003); Kono et al., Hum. Mol. Genet. 14: 2881-2892 (2005); and Kyogoju et al., Arthritis Rheum. 46: 1242-1254 (2002)), and every genetic polymorphism that will be reported in the future.

In FcγRIIa, there are two allotypes, one where the amino acid at position 131 of FcγRIIa is histidine (type H) and the other where the amino acid at position 131 is substituted with arginine (type R) (Warrmerdam, J. Exp. Med. 172: 19-25 (1990)).

The FcγR includes human, mouse, rat, rabbit, and monkey-derived FcγRs but is not limited thereto, and may be derived from any organism. Mouse FcγRs include FcγRI (CD64), FcγRII (CD32), FcγRIII (CD16), and FcγRIII-2 (CD 16-2), and any mouse FcγRs, or FcγR isoforms, but are not limited thereto.

The amino acid sequence of human FcγRI is set forth in NP_000557.1; the amino acid sequence of human FcγRIIa is set forth in AAH20823.1 and such; the amino acid sequence of human FcγRIIb is set forth in AAI46679.1 and such; the amino acid sequence of human FcγRIIIa is set forth in AAH33678.1 and such; and the amino acid sequence of human FcγRIIIb is set forth in AAI28563.1.

Unlike Fcγ receptor belonging to the immunoglobulin superfamily, human FcRn is structurally similar to polypeptides of major histocompatibility complex (MHC) class I, exhibiting 22% to 29% sequence identity to class I MHC molecules (Ghetie et al., Immunol. Today (1997) 18 (12): 592-598). FcRn is expressed as a heterodimer consisting of soluble β chain or light chain (β2 microglobulin) complexed with transmembrane α chain or heavy chain. Like MHC, FcRn α chain comprises three extracellular domains (α1, α2, and α3) and its short cytoplasmic domain anchors the protein onto the cell surface. α1 and α2 domains interact with the FcRn-binding domain of the antibody Fc region (Raghavan et al., Immunity (1994) 1: 303-315). The amino acid sequence of human FcRn is set forth in NP_004098.1, and the amino acid sequence of β2 microglobulin is set forth in NP_004039.1.

A "parent Fc region" as used herein refers to an Fc region prior to introduction of amino acid alteration(s) described herein. In some embodiments, the parent Fc region is an Fc region of a native sequence (or an Fc region of a native antibody). An antibody includes, for example, IgA (IgA1, IgA2), IgD, IgE, IgG (IgG1, IgG2, IgG3, IgG4), and IgM. An antibody may be derived from human or monkey (e.g., cynomolgus, rhesus macaque, marmoset, chimpanzee, or baboon). A native antibody may include naturally-occurring mutations. A plurality of allotype sequences of IgGs due to genetic polymorphism are described in "Sequences of proteins of immunological interest", NIH Publication No. 91-3242, and any of them may be used in the present invention. In particular, for human IgG1, the amino acid sequence at positions 356 to 358 (EU numbering) may be either DEL or EEM. In addition, for human IgG1, the amino acid at position 214 (EU numbering) may be either K or R. In certain embodiments, the parent Fc region is an Fc region derived from a heavy chain constant region of human IgG1, human IgG2, human IgG3, or human IgG4. In another embodiment, the parent Fc region is an Fc region derived from a heavy chain constant region of SEQ ID NO: 1, 28, 29, or 56. In further embodiments, the parent Fc region may be an Fc region produced by adding an amino acid alteration(s) other than the amino acid alteration(s) described herein to an Fc region of a native sequence (an Fc region of reference variant sequence). An Fc region of a native sequence is generally constituted as a homodimer consisting of two identical polypeptide chains.

In addition, amino acid alterations performed for other purpose(s) can be combined in a variant Fc region described herein. For example, amino acid substitutions that improve FcRn-binding activity (Hinton et al., J. Immunol. 176(1): 346-356 (2006); Dall'Acqua et al., J. Biol. Chem. 281(33): 23514-23524 (2006); Petkova et al., Intl. Immunol. 18(12): 1759-1769 (2006); Zalevsky et al., Nat. Biotechnol. 28(2): 157-159 (2010); WO 2006/019447; WO 2006/053301; and WO 2009/086320), and amino acid substitutions for improving antibody heterogeneity or stability (WO 2009/041613) may be added. Alternatively, polypeptides with the property of promoting antigen clearance, which are described in WO 2011/122011, WO 2012/132067, WO 2013/046704 or WO 2013/180201, polypeptides with the property of specific binding to a target tissue, which are described in WO 2013/180200, polypeptides with the property of repeated binding to a plurality of antigen molecules, which are described in WO 2009/125825, WO 2012/073992 or WO 2013/047752, can be combined with a variant Fc region described herein. Alternatively, with the objective of conferring binding ability to other antigens, the amino acid alterations disclosed in EP1752471 and EP1772465 may be combined in CH3 of a variant Fc region described herein. Alternatively, with the objective of increasing plasma retention, amino acid alterations that decrease the pI of the constant region (WO 2012/016227) may be combined in a variant Fc region described herein. Alternatively, with the objective of promoting uptake into cells, amino acid alterations that increase the pI of the constant region (WO 2014/145159) may be combined in a variant Fc region described herein. Alternatively, with the objective of promoting elimination of a target molecule from plasma, amino acid alterations that increase the pI of the constant region (WO 2016/125495) may be combined in a variant Fc region described herein. In one embodiment, such alteration may include, for example, substitution at at least one position selected from the group consisting of positions 311, 343, 384, 399, 400, and 413 according to EU numbering. In a further embodiment, such substitution may be a replacement of an amino acid with Lys or Arg at each position.

Methods for producing heterodimerized antibodies are not limited to these, but such antibodies may be produced by the knob-in-hole technology (see, for example, Nat. Biotechnol., (16);677-681 (1998) and U.S. Patent No. 5,731,168) or by engineering electrostatic steering effects (WO2006/106905, WO2009/089004A1, J. Biol. Chem., (285), 19637-19646 (2010), etc.).

For association of heterologous polypeptides comprising variant Fc regions, a technique of suppressing unintended association of homologous polypeptides comprising variant Fc regions by introducing electrostatic repulsion into the interface of the CH2 or CH3 domain of the Fc region can be applied, as described in WO 2006/106905.

Examples of amino acid residues in contact at the interface of the CH2 or CH3 domain of the Fc region include the residue at position 356 (EU numbering), the residue at position 439 (EU numbering), the residue at position 357 (EU numbering), the residue at position 370 (EU numbering), the residue at position 399 (EU numbering), and the residue at position 409 (EU numbering) in the CH3 domain.

More specifically, for example, the Fc region in which one to three pairs of amino acid residues selected from (1) to (3) shown below have the same charge can be produced: (1) amino acid residues at positions 356 and 439 (EU numbering) in the CH3 domain; (2) amino acid residues at positions 357 and 370 (EU numbering) in the CH3 domain; and (3) amino acid residues at positions 399 and 409 (EU numbering) in the CH3 domain.

Furthermore, heterologous polypeptides comprising variant Fc regions can be produced, wherein one to three pairs of amino acid residues selected from (1) to (3) indicated above have the same charge in the CH3 domain of the first Fc region, and the pairs of amino acid residues selected in the aforementioned first Fc region also have the same charge in the CH3 domain of the second Fc region, provided that the charges in the first and second Fc regions are opposite.

In the above-mentioned Fc regions, for example, negatively-charged amino acid residues are preferably selected from glutamic acid (E) and aspartic acid (D), and positively-charged amino acid residues are preferably selected from lysine (K), arginine (R), and histidine (H).

Other known techniques can be used additionally for association of heterologous polypeptides comprising variant Fc regions. Specifically, such a technique is conducted by substituting an amino acid side chain present in one of the Fc regions with a larger side chain (knob; which means "bulge"), and substituting an amino acid side chain present in the Fc region with a smaller side chain (hole; which means "void"), to place the knob within the hole. This can promote efficient association between Fc-region-containing polypeptides having different amino acid sequences from each other (WO 1996/027011; Ridgway et al., Prot. Eng. 9:617-621 (1996); Merchant et al., Nat.Biotech. 16, 677-681 (1998)).

In addition, other known techniques can also be used for heterologous association of polypeptides comprising variant Fc regions. Association of polypeptides comprising an Fc region can be induced efficiently using strand-exchange engineered domain CH3 heterodimers (Davis et al., Prot. Eng. Des. & Sel., 23:195-202 (2010)). This technique can also be used to efficiently induce association between Fc region-containing polypeptides having different amino acid sequences.

In addition, heterodimerized antibody production techniques that use association of antibody CH1 and CL, and association of VH and VL, which are described in WO 2011/028952, can also be used.

As with the method described in WO 2008/119353 and WO 2011/131746, it is also possible to use the technique of producing heterodimerized antibodies by producing two types of homodimerized antibodies in advance, incubating the antibodies under reducing conditions to dissociate them, and allowing them to associate again.

Furthermore, as with the method described in WO 2012/058768, it is also possible to use the technique of producing heterodimerized antibodies by adding alterations to the CH2 and CH3 domains.

When simultaneously expressing two polypeptides comprising a variant Fc region which have different amino acid sequences, in order to produce polypeptides comprising heterologous variant Fc regions, polypeptides comprising homologous variant Fc regions are also usually produced as impurities. In such cases, polypeptides comprising heterologous variant Fc regions can be efficiently obtained by separating and purifying them from polypeptides comprising homologous variant Fc regions using known technologies. A method has been reported to efficiently separate and purify heterodimerized antibodies from a homodimerized antibodies using ion exchange chromatography, by introducing amino acid alterations into the variable regions of the two types of antibody heavy chains to create a difference in isoelectric points between the homodimerized antibodies and the heterodimerized antibodies (WO 2007/114325). Another method has been reported to purify heterodimerized antibodies using Protein A chromatography, by constructing a heterodimerized antibody comprising two types of heavy chains derived from mouse IgG2a that binds to Protein A and rat IgG2b that does not bind to Protein A (WO 1998/050431 and WO 1995/033844).

Furthermore, a heterodimerized antibody can be efficiently purified using Protein A chromatography, by substituting amino acid residues at positions 435 and 436 (EU numbering), which are located in the Protein A binding site of an antibody heavy chain, with amino acids such as Tyr or His, to yield different Protein A binding affinities.

In the present invention, amino acid alteration means any of substitution, deletion, addition, insertion, and modification, or a combination thereof. In the present invention, amino acid alteration may be rephrased as amino acid mutation.

The number of amino acid alterations introduced into an Fc region is not limited. In certain embodiments, it can be 1, 2 or less, 3 or less, 4 or less, 5 or less, 6 or less, 8 or less, 10 or less, 12 or less, 14 or less, 16 or less, 18 or less, 20 or less, 22 or less, 24 or less, 26 or less, 28 or less, or 30 or less.

In one aspect, the present invention provides methods of producing a polypeptide comprising a variant Fc region. In further aspects, the present invention provides methods of producing a polypeptide comprising a variant Fc region whose function has been modified. In further aspects, the present invention provides methods for modifying a function of a polypeptide comprising an Fc region. In some aspects, the polypeptides are antibodies. In some aspects, the polypeptides are Fc fusion proteins. In certain embodiments, those methods comprise introducing at least one amino acid alteration into the parent Fc region. In certain embodiments, those methods comprise: (i) providing a polypeptide(s) comprising the parent Fc region; and (ii) introducing at least one amino acid alteration into the parent Fc region. In certain embodiments, those methods may further comprise (iii) measuring the function of the polypeptide(s) comprising the variant Fc region. A native Fc region is usually composed of two identical polypeptide chains. Amino acid alterations to the parent Fc region may be introduced into either one of the two polypeptide chains of the parent Fc region, or into both of the two polypeptide chains.

In another embodiment, the method of producing polypeptide(s) comprising a variant Fc region comprises: (i) providing one or more nucleic acids encoding polypeptides comprising the parent Fc region; (ii) introducing at least one mutation into the region(s) encoding the parent Fc region in the nucleic acids; (iii) introducing the nucleic acids produced in (ii) into a host cell; and (iv) culturing the cell described in (iii) such that the polypeptide(s) comprising the variant Fc region are expressed. In certain embodiments, the above methods may further comprise (v) collecting the polypeptide(s) comprising the variant Fc region from the host cell culture described in (iv).

In certain embodiments, the nucleic acids produced in (ii) may be included in one or more vectors (*e.g.*, expression vectors).

In some embodiments, the amino acid alterations used in the production methods of the present invention are selected from any single alteration selected from among the amino acid alterations that can be comprised in the above-mentioned variant Fc regions, combinations of the single alterations, or the combined alterations listed in Table 1.

An Fc region may be obtained by re-eluting the fraction adsorbed onto Protein A column after partially digesting IgG1, IgG2, IgG3, IgG4 monoclonal antibodies or such using a protease such as pepsin. The protease is not particularly limited as long as it can digest a full-length antibody so that Fab and F(ab')2 are produced in a restrictive manner by appropriately setting the enzyme reaction conditions such as pH, and examples include pepsin and papain.

The polypeptides comprising a variant Fc region of the present invention may be produced by other methods known in the art in addition to the above-mentioned production methods. The polypeptides comprising a variant Fc region produced by the production methods described herein are also included in the present invention.

In one embodiment, isolated nucleic acid encoding a polypeptide comprising a variant Fc region of the present invention is provided. Such nucleic acid may encode an amino acid sequence comprising the first polypeptide of the variant Fc region and/or an amino acid sequence comprising the second polypeptide of the variant Fc region. In a further embodiment, one or more vectors (*e.g.*, expression vectors) comprising such nucleic acid are provided. In a further embodiment, a host cell comprising such nucleic acid is provided. In one such embodiment, a host cell comprises (*e.g.*, has been transformed with): (1) a vector comprising a nucleic acid that encodes an amino acid sequence comprising the first polypeptide of the variant Fc region and an amino acid sequence comprising the second polypeptide of the variant Fc region, or (2) a first vector comprising a nucleic acid that encodes an amino acid sequence comprising the first polypeptide of the variant Fc region and a second vector comprising a nucleic acid that encodes an amino acid sequence comprising the second polypeptide of the variant Fc region. In one embodiment, the host cell is eukaryotic, *e.g.*, a Chinese Hamster Ovary (CHO) cell or lymphoid cell *(e.g.,* Y0, NS0, Sp2/0 cell). In one embodiment, a method of making a polypeptide comprising a variant Fc region of the present invention is provided, wherein the method comprises culturing a host cell comprising a nucleic acid encoding the polypeptide comprising a variant Fc region of the present invention under conditions suitable for expression of the polypeptide , and optionally recovering the polypeptide from the host cell (or host cell culture medium).

For recombinant production of a polypeptide comprising a variant Fc region of the present invention, nucleic acid encoding the polypeptide is isolated and inserted into one or more vectors for further cloning and/or expression in a host cell. Such nucleic acid may be readily isolated and sequenced using conventional procedures (*e.g.*, by using oligonucleotide probes that are capable of binding specifically to genes encoding the Fc region of the antibody).

Suitable host cells for cloning or expression of vectors encoding a polypeptide comprising a variant Fc region of the present invention include prokaryotic or eukaryotic cells.

In addition to prokaryotes, eukaryotic microbes such as filamentous fungi or yeast are suitable cloning or expression hosts for vectors encoding a polypeptide comprising a variant Fc region of the present invention, including fungi and yeast strains whose glycosylation pathways have been "humanized," resulting in the production of an antibody Fc region with a partially or fully human glycosylation pattern. See Gerngross, Nat. Biotech. 22: 1409-1414 (2004), and Li et al., Nat. Biotech. 24: 210-215 (2006).

Suitable host cells for the expression of glycosylated antibody Fc region are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. *See, e.g.*, US Patent Nos. 5,959,177, 6,040,498, 6,420,548, 7,125,978, and 6,417,429.

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, *e.g.,* in Graham et al., J. Gen Virol. 36: 59 (1977)); baby hamster kidney cells (BHK); mouse sertoli cells (TM4 cells as described, *e.g.,* in Mather, Biol. Reprod. 23: 243-251 (1980)); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor (MMT 060562); TRI cells, as described, *e.g.,* in Mather et al., Annals N.Y. Acad. Sci. 383: 44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77: 4216 (1980)); and myeloma cell lines such as Y0, NS0 and Sp2/0.

The assay methods described herein or various measurement methods known in the art may be used for identifying or screening for the variant Fc regions provided herein, or for elucidating their physical or chemical properties or biological activities.

Assays for determining the binding activity of a polypeptide containing a variant Fc region towards one or more FcR family members are described herein or otherwise known in the art. Such binding assays include but are not limited to surface plasmon resonance assay, Amplified Luminescent Proximity Homogeneous Assay (ALPHA) screening, ELISA, and fluorescence activated cell sorting (FACS) (Lazar et al., Proc. Natl. Acad. Sci. USA (2006) 103(11): 4005-4010).

In one embodiment, the binding activity of the polypeptide comprising a variant Fc region to FcR family members can be measured using a surface plasmon resonance assay. For example, various FcRs are subjected to interaction as analytes with a polypeptide comprising a variant Fc region immobilized or captured onto a sensor chip by using known methods and reagents (e.g., Protein A, Protein L, Protein A/G, Protein G, anti-λ chain antibodies, anti-κ chain antibodies, antigenic peptides, and antigenic proteins). Alternatively, FcRs may be immobilized or captured onto a sensor chip, and a polypeptide comprising a variant Fc region may be used as an analyte. As a result of such interactions, binding sensorgrams are obtained and by analyzing them, the dissociation constant (KD) values of such bindings can be calculated. Moreover, the difference in the resonance unit (RU) value in sensorgrams before and after being subjected to interaction with an FcR (*i.e.*, the binding amount of the FcR) can be used as an indicator of the binding activity of a polypeptide comprising a variant Fc region to the FcR. Furthermore, the corrected value obtained by dividing the above-mentioned binding amount of the FcR by the difference in the RU value in the sensorgrams before and after the polypeptide comprising a variant Fc region are immobilized or captured onto the sensor chip (*i.e.*, the binding amount of the polypeptide comprising a variant Fc region) (that is, the correction value is the binding amount of the FcR per unit amount of the polypeptide comprising a variant Fc region) may be used as an indicator of the binding activity.

Any of the polypeptides comprising a variant Fc region provided herein may be used in therapeutic methods.

In one aspect, a polypeptide comprising a variant Fc region for use as a medicament is provided. In further aspects, a polypeptide comprising a variant Fc region for use in treating tumor is provided. In certain embodiments, a polypeptide comprising a variant Fc region for use in a method of treatment is provided. In certain embodiments, the present invention provides a polypeptide comprising a variant Fc region for use in a method of treating an individual having tumor comprising administering to the individual an effective amount of the polypeptide comprising a variant Fc region. In one such embodiment, the method further comprises administering to the individual an effective amount of at least one additional therapeutic agent. In further embodiments, the present invention provides a polypeptide comprising a variant Fc region for use in damaging cells. In certain embodiments, the present invention provides a polypeptide comprising a variant Fc region for use in a method of damaging cells in an individual comprising administering to the individual an effective amount of the polypeptide comprising a variant Fc region to damage cells. An "individual" according to any of the above embodiments is preferably a human.

In some embodiments, the tumor is a solid tumor. In a solid tumor, tumor cells usually proliferate to form a population, and the tumor tissue is formed mainly by these cells. Furthermore, tumor tissues in living organisms are often infiltrated by immune cells such as lymphocytes, which also constitute part of tumor tissues. In one embodiment, damage to cells is elicited by ADCC activity, CDC activity, or ADCP activity.

In a further aspect, the present invention provides for the use of a polypeptide comprising a variant Fc region in the manufacture or preparation of a medicament. In one embodiment, the medicament is for treatment of tumor. In a further embodiment, the medicament is for use in a method of treating tumor comprising administering to an individual having tumor an effective amount of the medicament. In one such embodiment, the method further comprises administering to the individual an effective amount of at least one additional therapeutic agent. In a further embodiment, the medicament is for damaging cells. In a further embodiment, the medicament is for use in a method of damaging cells in an individual comprising administering to the individual an effective amount of the medicament to damage cells. An "individual" according to any of the above embodiments may be a human.

In a further aspect, the present invention provides a method for treating a tumor. In one embodiment, the method comprises administering to an individual having such tumor an effective amount of a polypeptide comprising a variant Fc region. In one such embodiment, the method further comprises administering to the individual an effective amount of at least one additional therapeutic agent. An "individual" according to any of the above embodiments may be a human.

In a further aspect, the present invention provides a method for damaging cells in an individual. In one embodiment, the method comprises administering to the individual an effective amount of a polypeptide comprising a variant Fc region to damage cells. In one embodiment, an "individual" is a human.

In a further aspect, the present invention provides pharmaceutical formulations (pharmaceutical compositions) comprising any of the polypeptides comprising a variant Fc region provided herein. In one embodiment, the above-mentioned pharmaceutical formulation (pharmaceutical composition) further comprises a pharmaceutically acceptable carrier. In one embodiment, the present invention provides a pharmaceutical formulation (pharmaceutical composition) for use in treating tumor. In one embodiment, the present invention provides a pharmaceutical formulation (pharmaceutical composition) for use in damaging cells. In another embodiment, a pharmaceutical formulation (pharmaceutical composition) comprises a polypeptide comprising a variant Fc region provided herein and at least one additional therapeutic agent.

[Examples]

[Example 1] Production of Fc region variants with enhanced FcγR-binding ability

Although Fc variants for enhancing cytotoxic effector functions ADCC and ADCP were previously reported, variants with symmetrically engineered CH2 domains and low-fucose antibodies made by sugar chain modifications all still had room for further enhancement of FcγR binding. In addition, although the variants with asymmetrically engineered CH2 domains described in WO2013002362 and WO2014104165 had greatly enhanced FcγR-binding ability compared to symmetrically-engineered Fc region variants, they had room for further improvement. Specifically, the Fc region variant Kn125/H1076 described in WO2013002362 and WO2014104165 (abbreviated name herein: ART1) had a strongly enhanced binding ability to FcγRIIIa, but its FcγRIIa-binding ability was enhanced only a few times compared to IgG1, so further enhancement seemed necessary to exhibit strong ADCP activity. Kn120/H1068 (abbreviated name herein: ART2) had enhanced binding to both FcγRIIa and FcγRIIIa and was expected to show strong ADCC and ADCP activity. However, its binding ability to inhibitory FcγRIIb was also enhanced, and therefore its A/I ratio, an index for exertion of excellent effector functions, was low. Thus, the existing methods have not achieved the most ideal profile, i.e. "antibody modification technology for stronger binding to activating FcγRIIa and FcγRIIIa and suppressed binding to inhibitory FcγRIIb". Accordingly, in the present invention, further combinations of modifications were studied, and the production of Fc region variants with a superior profile that would overcome the above-mentioned problems was studied.

Existing Fc region variants to be used as references for comparison were prepared as follows: First, antibody heavy chain gene H240-G1d (SEQ ID NO: 1) as described in WO2014104165, which has a heavy chain variable region against human Epiregulin and a heavy chain constant region sequence of human IgG1, was produced. G1d is a sequence obtained by deleting C-terminal Lys and Gly from the heavy chain constant region sequence of native human IgG1. Knobs-into-holes modifications (Nat. Biotechnol., 1998, 16, 677), which are modifications for promoting heterodimerization, were introduced into the CH3 domain of H240-G1d, and modifications for enhancing FcγR binding were asymmetrically introduced into the CH2 domain, to produce Fc region variants to be used as references for comparison, ART1 and ART2. ART1, an Fc region variant with enhanced FcγRIIIa binding described in WO2013002362 and WO2014104165, was produced as follows: modifications for enhancing FcγR binding, L234Y/L235Q/G236W/S239M/H268D/D270E/S298A, were introduced into the CH2 domain of H240-G1d, and Y349C/T366W were introduced into the CH3 domain, to produce H240-Kn125 (SEQ ID NO: 2). In addition, D270E/K326D/A330M/K334E were introduced into the CH2 domain of H240-G1d, and D356C/T366S/L368A/Y407V were introduced into the CH3 domain, to produce H240-H1076 (SEQ ID NO: 3). Plasmids containing H240-Kn125, H240-H1076, and the gene for the light chain of anti-human Epiregulin antibody, L73-k0 (SEQ ID NO: 4), were mixed and introduced into the human embryonic kidney cell-derived Expi293 cell line (Invitrogen) by lipofection. After 4 days of culture, the supernatant was subjected to purification by a method known to those skilled in the art using rProtein A Sepharose™ Fast Flow (Amersham Biosciences) to obtain an Fc variant antibody against human Epiregulin (H240-Kn125/L73-k0//H240-H1076/L73-k0, abbreviated antibody name: EGL-ART1). The 280 nm absorbance of the purified antibody solution was measured using a spectrophotometer. The concentration of the purified antibody was calculated from the obtained measurement value using an extinction coefficient calculated by the PACE method (Protein Science 1995; 4: 2411-2423).

Similarly, an Fc region variant with enhanced binding to both FcγRIIa and FcγRIIIa described in WO2013002362 and WO2014104165, EGL-ART2 (H240-Kn120/L73-k0//H240-H1068/L73-k0), was produced. Further, different combinations of FcγR binding-enhancing modifications, G236A, S239D, A330L, and I332E, were symmetrically introduced into the CH2 domain to produce known FcγR binding-enhanced antibodies, EGL-SDALIE (H240-Kn032/L73-k0//H240-H1032/L73-k0), EGL-GASDIE (H240-Kn037/L73-k0//H240-H1036/L73-k0), and EGL-GASDALIE (H240-GASDALIE/L73-k0). In addition to these, an afucosylated antibody, EGL-afucosyl, which reportedly has enhanced binding to FcγRIIIa (Glycobiol. Vol17 no1 pp. 104-118 (2006) and such), was produced as a reference for comparison. In cells in which the expression of the fucose transporter gene is artificially suppressed on both homologous chromosomes, the function of the fucose transporter is inhibited. These cells can be used to obtain fucose-deficient antibodies (WO2006/067913 and such). Fucose-deficient antibodies can also be obtained by producing antibodies in cells forced to express beta 1, 4-N-acetylglucosaminyltransferase III and Golgi alpha-mannosidase II (Biotechnol, Bioeng. (2006) 93 (5), 851-861). Using these methods known to those skilled in the art, EGL-afucosyl (H240-G1d/L73-k_glycomab) was produced.

In order to produce Fc region variants superior to these existing variants, the new Fc region variants shown in Table 1, namely, ART3, ART4, ART5, ART6, ART8, ART10, ART11, and ART12, were produced. These variants all had L234F, L235Q, G236W, S239M, H268D, D270E, and S298A introduced into one of the heavy chains, and D270E, S298A, K326D, and 334E introduced into the other heavy chain, and were produced by introducing, in addition to these core asymmetrical modifications, a combination of modifications for altering the FcγR binding. Specifically, for the chain to which L234F/L235Q/G236W/S239M/H268D/D270E/S298A were introduced, further introduction of K326D, A330M, and I332E was examined. For the chain to which D270E/S298A/K326D/K334E were introduced, further introduction of G236A, I332E, I332D, and A330M was examined. In addition to these FcγR binding-enhancing modifications, T250V and T307P, which are modifications for improving antibody stability as described in WO2013118858, were introduced into both chains of ART4, ART5, ART6, ART8, ART10, ART11, and ART12.

The correspondences between the names of heavy chain constant regions used here and SEQ ID NOs are as follows: G1d (SEQ ID NO: 29), Kn125 (SEQ ID NO: 30), H1076 (SEQ ID NO: 31), k0 (SEQ ID NO: 32), Kn120 (SEQ ID NO: 33), H1068 (SEQ ID NO: 34), Kn443 (SEQ ID NO: 35), H1408 (SEQ ID NO: 36), Kn456 (SEQ ID NO: 37), H1446 (SEQ ID NO: 38), Kn462 (SEQ ID NO: 39), H1441 (SEQ ID NO: 40), Kn462 (SEQ ID NO: 41), H1445 (SEQ ID NO: 42), Kn461 (SEQ ID NO: 43), H1443 (SEQ ID NO: 44), Kn494 (SEQ ID NO: 45), H1514 (SEQ ID NO: 46), Kn496 (SEQ ID NO: 47), H1516 (SEQ ID NO: 48), Kn498 (SEQ ID NO: 49), H1518 (SEQ ID NO: 50), GASDALIE (SEQ ID NO: 51), Kn032 (SEQ ID NO: 52), H1032 (SEQ ID NO: 53), Kn037 (SEQ ID NO: 54), H1036 (SEQ ID NO: 55), G4d (SEQ ID NO: 56).

(Table 1) Produced Fc region variants and introduced modifications

| Abbreviated name of antibody | Heavy chain name | SEQ ID NO: | Modifications introduced into CH2 domain of G1d | Modifications introduced into CH3 domain of G1d |
|---|---|---|---|---|
| EGL-G1d | H240-G1d | 1 | | |
| EGL-ART1 | H240-Kn125 | 2 | L234Y/L235Q/G236W/S239M/H268D/D270E/S298A | Y349C/T366W |
| | H240-HI076 | 3 | D270E/K326D/A330M/K334E | D356C/T366S/L368A/Y407V |
| EGL-ART2 | H240-Kn120 | 5 | L234Y/L235Y/G236W/S239M/H268D/S298A/A327D | Y349C/T366W |
| | H240-H1068 | 6 | D270E/K326D/A330K/K334E | D356C/T366S/L368A/Y407V |
| EGL-ART3 | H240-Kn443 | 7 | L234F/L235Q/G236W/S239M/H268D/D270E/S298A/K326D | Y349C/T366W |
| | H240-H1408 | 8 | G236A/D270E/S298A/K326D/K334E | D356C/T366S/L368A/Y407V |
| EGL-ART4 | H240-Kn456 | 9 | L234F/L235Q/G236W/S239M/T250V/H268D/D270E/S298A/T307P/A330M/I332E | Y349C/T366W |
| | H240-H1446 | 10 | G236A/T250V/D270E/S298A/T307P/K326D/A330M/I332E/K334E | D356C/T366S/L368A/Y407V |
| EGL-ART5 | H240-Kn462 | 11 | L234F/L235Q/G236W/S239M/T250V/H268D/D270E/S298A/T307P/K326D | Y349C/T366W |
| | H240-H1441 | 12 | G236A/T250V/D270E/S298A/T307P/K326D/K334E | D356C/T366S/L368A/Y407V |
| EGL-ART6 | H240-Kn462 | 13 | L234F/L235Q/G236W/S239M/T250V/H268D/D270E/S298A/T307P/K326D | Y349C/T366W |
| | H240-H1445 | 14 | G236A/T250V/D270E/S298A/T307P/K326D/A330K/I332D/K334E | D356C/T366S/L368A/Y407V |
| EGL-ART8 | H240-Kn461 | 15 | L234F/L235Q/G236W/S239M/T250V/H268D/D270E/S298A/T307P/K326D/I332E | Y349C/T366W |
| | H240-H1443 | 16 | G236A/T250V/D270E/S298A/T307P/K326D/I332E/K334E | D356C/T366S/L368A/Y407V |

(continued)

| Abbreviated name of antibody | Heavy chain name | SEQ ID NO: | Modifications introduced into CH2 domain of G1d | Modifications introduced into CH3 domain of G1d |
|---|---|---|---|---|
| EGL-ART10 | H240-Kn494 | 17 | L234F/L235Q/G236W/S239M/T250V/H268D/D270E/S298A/T307P/I332E | Y349C/T366W |
| | H240-H1514 | 18 | T250V/D270E/S298A/T307P/K326D/1332E/K334E | D356C/T366S/L368A/Y407V |
| EGL-ART11 | H240-Kn496 | 19 | L234F/L235Q/G236W/S239M/T250V/H268D/D270E/S298A/T307P | Y349C/T366W |
| | H240-H1516 | 20 | T250V/D270E/S298A/T307P/K326D/K334E | D356C/T366S/L368A/Y407V |
| EGL-ART12 | H240-Kn498 | 21 | L234F/L235Q/G236W/S239M/T250V/H268D/D270E/S298A/T307P/A330M | Y349C/T366W |
| | H240-H1518 | 22 | T250V/D270E/S298A/T307P/K326D/A330M/K334E | D356C/T366S/L368A/Y407V |
| EGL-GASDALIE | **H240-GASDALIE** | 23 | G236A/S239D/A330L/I332E | |
| EGL-SDALIE | **H240-Kn032** | 24 | S239D/A330L/I332E | Y349C/T366W |
| | **H240-HI032** | 25 | S239D/A330L/I332E | D356C/T366S/L368A/Y407V |
| EGL-GASDIE | **H240-Kn037** | 26 | G236A/S239D/I332E | Y349C/T366W |
| | **H240-HI036** | 27 | G236A/S239D/I332E | D356C/T366S/L368A/Y407V |
| EGL-afucosyl | **H240-G1d** | 1 | | |

[Example 2] Evaluation of FcγR binding of Fc region variants

The extracellular domains of FcγRs were produced by the method described in WO2014104165. The interaction between the produced antibodies and human FcγRs was analyzed using Biacore 8K+ by the method below. For the running buffer, 50 mM Na-Phosphate, 150 mM NaCl, and 0.05% Tween20 (pH 7.4) was used, and measurement was performed at 25°C. The sensor chip used was a Series S SA chip (GE Healthcare) onto which CaptureSelect Human Fab-kappa Kinetics Biotin Conjugate (Thermo Fisher Scientific) was immobilized. An antibody of interest was captured onto this chip, and each FcγR diluted with the running buffer was allowed to interact with it. The chip was regenerated using 10 mM Glycine-HCl (pH 1.5), and repeatedly used to capture antibodies and perform measurement. The dissociation constants KD (mol/L) of each antibody for FcγRs were calculated using Biacore Insight Evaluation Software, with a steady state affinity model for the dissociation constant for FcγRIIb and a 1:1 Langmuir binding model for the dissociation constants for the other FcγRs (Table 2).

(Table 2) Measurement of binding between produced variants and human FcγRs

| Abbreviated name of constant region | Antibody name | $K_D$ (M) for hFcγRs | | | | | | Relative value to $K_D$ between G1d and hFcgRs | | | | | | A/I ratio ($K_D$ for FcγRIIb divided by $K_D$ for each FcγR) | | | |
|---|---|---|---|---|---|---|---|---|---|---|---|---|---|---|---|---|---|
| | | hFcγR Ia | hFcγR IIa H | hFcγR IIa R | hFcγR IIb | hFcγR IIIa F | hFcγR IIIa V | hFcγR Ia | hFcγR IIa H | hFcγR IIa R | hFcγR IIb | hFcγR IIIa F | hFcγR IIIa V | hFcγR IIa H | hFcγR IIa R | hFcγR IIIa F | hFcγR IIIa V |
| G1d | H 240-G 1 d/U 3-k0 | 9.01E-11 | 1.12E-06 | 1.66E-06 | 1.01E-05 | 2.96E-06 | 3.58E-07 | 1.0 | 1.0 | 1.0 | 1.0 | 1.0 | 1.0 | 8.9 | 6.1 | 3.4 | 28.1 |
| ART1 | H240-Kn125/L73-k0//H240-H1076/L73-k0 | 3.02E-11 | 2.64E-07 | 1.01E-06 | 1.25E-05 | 2.53E-09 | 1.11E-09 | 3.0 | 4.3 | 1.6 | 0.8 | 1170.2 | 322.8 | 47.4 | 12.4 | 4947.2 | 11261.3 |
| ART2 | H240-Kn120/L73-k0//H240-H1068/L73-k0 | 2.64E-11 | 7.64E-08 | 3.38E-08 | 4.40E-07 | 6.43E-09 | 1.67E-09 | 3.4 | 14.7 | 49.0 | 22.8 | 459.6 | 214.6 | 5.8 | 13.0 | 68.4 | 263.7 |
| ART3 | H240-Kn443/L73-k0//H240-HI408/L73-k0 | 1.72E-10 | 3.50E-08 | 1.21E-07 | 6.33E-06 | 1.60E-08 | 3.16E-09 | 0.5 | 32.1 | 13.7 | 1.6 | 185.2 | 113.5 | 180.7 | 52.4 | 396.1 | 2003.7 |
| ART4 | H240-Kn456/L73-k0//H240-H1446/L73-k0 | 3.05E-11 | 1.79E-07 | 5.18E-07 | 1.17E-05 | 1.17E-09 | 7.75E-10 | 2.9 | 6.3 | 3.2 | 0.9 | 2519.9 | 462.2 | 65.2 | 22.5 | 9943.2 | 15047.3 |
| ART5 | H240-Kn462/L73-k0//H240-H1441/L73-k0 | 1.69E-10 | 3.32E-08 | 1.60E-07 | 7.96E-06 | 2.00E-08 | 3.86E-09 | 0.5 | 33.9 | 10.4 | 1.3 | 148.1 | 92.9 | 240.1 | 49.9 | 398.8 | 2064.8 |
| ART6 | H240-Kn462/L73-k0//H240-H1445/L73-k0 | 7.37E-11 | 9.52E-09 | 6.89E-08 | 2.92E-06 | 3.00E-09 | 1.11E-09 | 1.2 | 118.0 | 24.1 | 3.4 | 986.7 | 321.9 | 307.0 | 42.4 | 975.5 | 2625.7 |

| Abbreviated name of constant region | Antibody name | $K_D$ (M) for hFcγRs | | | | | | Relative value to $K_D$ between G1d and hFcgRs | | | | | | A/I ratio ($K_D$ for FcγRIIb divided by $K_D$ for each FcγR) | | | |
|---|---|---|---|---|---|---|---|---|---|---|---|---|---|---|---|---|---|
| | | hFcγR Ia | hFcγR IIa H | hFcγR IIa R | hFcγR IIb | hFcγR IIIa F | hFcγR IIIa V | hFcγR Ia | hFcγR IIa H | hFcγR IIa R | hFcr R IIb | hFcγR IIIa F | hFcγR IIIa V | hFcr R IIa H | hFcγR IIa R | hFcγR IIIa F | hFcγR IIIa V |
| ART8 | H240-Kn461/L73-k0//H240-H1443/L73-k0 | 2.80E-11 | 7.49E-08 | 1.26E-07 | 3.53E-06 | 1.50E-09 | 5.16E-10 | 3.2 | 150 | 13.2 | 2.8 | 1966.7 | 694.9 | 47.2 | 28.1 | 2350.3 | 6852.0 |
| ART10 | H240-Kn494/L73-k0//H240-H1514/L73-k0 | 2.67E-11 | 4.20E-07 | 1.10E-06 | 7.25E-06 | 2.29E-09 | 6.34E-10 | 3.4 | 2.7 | 1.5 | 1.4 | 1289.2 | 565.5 | 17.3 | 6.6 | 3159.9 | 11436.1 |
| ART11 | H240-Kn496/L73-k0//H240-H1516/L73-k0 | 3.81E-11 | 2.31E-07 | 1.27E-06 | 1.46E-05 | 2.10E-08 | 3.67E-09 | 2.4 | 4.9 | 1.3 | 0.7 | 140.6 | 97.5 | 63.3 | 11.5 | 694.1 | 3974.6 |
| ART12 | H240-Kn498/L73-k0//H240-HI518/L73-k0 | 2.61E-11 | 3.66E-07 | 1.72E-06 | 2.21E-05 | 5.12E-09 | 2.27E-09 | 3.4 | 3.1 | 1.0 | 0.5 | 577.5 | 157.9 | 60.3 | 12.8 | 4309.9 | 9721.0 |
| GASDALIE | H240-GASDALIE/L73-k0 | 4.23E-12 | 1.62E-07 | 1.61E-07 | 2.67E-06 | 2.98E-08 | 1.52E-08 | 21.3 | 6.9 | 10.3 | 3.8 | 99.1 | 23.6 | 16.5 | 16.5 | 89.3 | 175.7 |
| SDALIE | H240-Kn032/L73-k0//H240-HI032/L73-k0 | 1.66E-12 | 1.18E-06 | 9.35E-07 | 2.59E-06 | 2.62E-08 | 9.73E-09 | 54.3 | 10 | 1.8 | 3.9 | 112.7 | 36.8 | 2.2 | 2.8 | 987 | 266.1 |
| GASDIE | H240-Kn037/L73-k0//H240-H1036/L73-k0 | 1.45E-11 | 6.86E-08 | 6.70E-08 | 1.25E-06 | 6.61E-08 | 2.38E-08 | 6.2 | 16.4 | 24.7 | 8.0 | 44.7 | 15.1 | 18.2 | 18.6 | 18.9 | 52.6 |

(continued)

| Abbreviated name of constant region | Antibody name | $K_D$ (M) for hFcγRs | | | | | | Relative value to $K_D$ between G1d and hFcgRs | | | | | | A/I ratio ($K_D$ for FcγRIIb divided by $K_D$ for each FcγR) | | | |
|---|---|---|---|---|---|---|---|---|---|---|---|---|---|---|---|---|---|
| | | hFcγR Ia | hFcγR IIa H | hFcγR IIa R | hFcγR IIb | hFcγR IIIa F | hFcγR IIIa V | hFcγR Ia | hFcγR IIa H | hFcγR IIa R | hFcrR IIb | hFcγR IIIa F | hFcγR IIIa V | hFcr R IIa H | hFcγR IIa R | hFcγR IIIa F | hFcγR IIIa V |
| Afucosyl | H240-G1d/L73-k_glycomab | 6.51E-11 | 9.99E-07 | 9.46E-07 | 5.65E-06 | 1.23E-07 | 1.90E-08 | 1.4 | 1.1 | 1.8 | 1.8 | 240 | 18.9 | 5.7 | 6.0 | 46.0 | 298.1 |

In the table, the "Relative value to KD between G1d and hFcγRs" is a value obtained by dividing the KD value of G1d for each FcγR by the KD value of each antibody for each FcγR, indicating how much each antibody was enhanced as compared to G1d. The "All ratio" is a value obtained by dividing the KD of each antibody for FcγRIIb by the KD for each FcγR, indicating how much the binding to activating FcγRs was selectively enhanced over the binding to inhibitory FcγR.

ART3, ART4, ART5, ART6, ART8, ART10, ART11, and ART12 produced in the present invention were all enhanced as compared to G1d for FcγRIIIaF and FcγRIIIaV. These variants were also more enhanced than the existing symmetrically-modified FcγR-enhanced antibodies GASDALIE, SDALIE, GASDIE, and Afucosyl antibody for both FcγRI-IIaF and FcγRIIIaV. Moreover, the FcγRIIIaF binding of ART4 (2519.9 times), ART6 (986.7 times), ART8 (1966.7 times), ART10 (1289.2 times), and ART12 (577.5 times) was more enhanced than that of ART2 (459.6 times), which is described in WO2014104165. Furthermore, the FcγRIIIaF binding of ART4, ART8, and ART10 was more enhanced even when compared to ART1 (1170.2 times), which binds to FcγRIIIaF more strongly than ART2. Similarly, for FcγRIIIaV, the binding of ART4 (462.2 times), ART6 (321.9 times), ART8 (694.9 times), and ART10 (565.5 times) was more enhanced than that of ART2 (214.6 times), and the binding of ART4, ART8, and ART10 was more enhanced even when compared to ART1 (322.8 times). The FcγRIIIaV binding of ART6 was comparable to that of ART1.

ART3 (32.1 times), ART4 (6.3 times), ART5 (33.9 times), ART6 (118.0 times), ART8 (15.0 times), ART10 (2.7 times), ART11 (4.9 times), and ART12 (3.1 times) were all enhanced as compared to G1d for FcγRIIaH. In particular, ART3, ART5, ART6, and ART8 were more enhanced even when compared to ART2 (14.7 times), an FcγRIIa-enhanced antibody with asymmetrically modified CH2 domains as described in WO2014104165. Moreover, ART3, ART5, and ART6 were more enhanced even when compared to the existing symmetrically-modified FcγRIIa-enhanced antibody GASDIE (16.4 times), and therefore are expected to show stronger ADCP activity than any existing variants. For the binding to FcγRIIaR, all of ART3 (13.7 times), ART4 (3.2 times), ART5 (10.4 times), ART6 (24.1 times), ART8 (13.2 times), ART10 (1.5 times), and ART11 (1.3 times) were enhanced as compared to G1d, but the existing variants GASDIE (24.7 times) and ART2 (49.0 times) were more enhanced than these variants. However, what should be noted here is selectivity for activating FcγRs. As the inhibitory receptor FcγRIIb induces intracellular signals that suppress immune responses in contrast to activating FcγRs, it is expected to inhibit signals from activating FcγRs. In fact, it has been reported that the anti-tumor effects of antibodies are increased in FcγRIIb knockout mice (Nature Medicine 2000, 6, 443-436). In addition, a correlation was observed between the anti-tumor effects and the ratios of binding to activating FcγR and inhibitory FcγR (A/I ratio) in each subclass of mouse IgG (Science 2005, 310, 1510-1512). Thus, in order to exert stronger effector functions, an antibody with enhanced binding to activating FcγRs and reduced binding to FcγRIIb may be necessary. However, the high sequence homology of FcγRIIaR with FcγRIIb makes it difficult to confer selectivity, and indeed it is hard to say that the variants previously reported have excellent selectivity. It was shown that the new variants produced in the present invention, ART10 (All ratio: 6.6), ART11 (A/I ratio: 11.5), ART12 (A/I ratio: 12.8), ART4 (A/I ratio: 22.5), ART8 (A/I ratio: 28.1), ART6 (All ratio: 42.4), ART5 (All ratio: 49.9), and ART3 (A/I ratio: 52.4), were all superior to G1d (All ratio: 6.1) in terms of the A/I ratio for FcγRIIaR, and among them, ART4, ART8, ART6, ART5, and ART3 were superior to ART2 (All ratio: 13.0) and GASDIE (All ratio: 18.6). Similarly, in terms of the All ratio for FcγRIIaH, ART10 (All ratio: 17.3), ART8 (A/I ratio: 47.2), ART12 (All ratio: 60.3), ART11 (All ratio: 63.3), ART4 (All ratio: 65.2), ART3 (All ratio: 180.7), ART5 (A/I ratio: 240.1), and ART6 (All ratio: 307.0) were superior to G1d (A/I ratio: 8.9), and among them, ART8, ART12, ART11, ART4, ART3, ART5, and ART6 showed better A/I ratios than ART2 (A/I ratio: 5.8) and GASDIE (All ratio: 18.2). From the above results, it can be said that ART4, ART8, ART3, ART5, and ART6 are antibodies that have better A/I ratios than the existing antibodies enhanced for FcγRIIaR. In addition, it can be said that ART3, ART5, and ART6 are antibodies that have better binding ability and A/I ratios than the existing antibodies enhanced for FcγRIIaH.

For FcγRIIIaF, the All ratios of ART3 (All ratio: 396.1), ART5 (A/I ratio: 398.8), ART11 (All ratio: 694.1), ART6 (A/I ratio: 975.5), ART8 (A/I ratio: 2350.3), ART10 (A/I ratio: 3159.9), ART12 (A/I ratio: 4309.9), and ART4 (A/I ratio: 9943.2) were all superior to that of G1d (A/I ratio: 3.4). Among them, ART4 showed a better A/I ratio than ART1 (All ratio: 4947.2), an FcγRIIIa specific enhanced variant described in WO2014104165. Similarly, for FcγRIIIaV, the All ratios of ART3 (All ratio: 2003.7), ART5 (A/I ratio: 2064.8), ART6 (All ratio: 2625.7), ART11 (All ratio: 3974.6), ART8 (A/I ratio: 6852.0), ART12 (A/I ratio: 9721.0), ART10 (A/I ratio: 11436.1), and ART4 (A/I ratio: 15047.3) were all better than those of G1d (A/I ratio: 28.1) and the existing enhance variant Afucosyl (All ratio: 298.1). Among them, ART4 and ART10 showed better All ratios than ART1 (A/I ratio: 11261.3), an FcγRIIIa specific enhanced variant described in WO2014104165. From the above results, it can be said that ART4 is an antibody that has more excellent binding ability and A/I ratios for both FcγRIIIaF and FcγRIIIaV as compared to the existing enhanced antibody ART1.

[Example 3] Evaluation of antibodies having modified Fc regions by ADCC reporter bioassay (3-1) Production of human epiregulin-expressing cells (Hepa1-6/hEREG)

Murine hepatoma cell line Hepa1-6 was purchased from ATCC. A human EREG (hEREG) gene was introduced into the cells by transfection, and constitutively expressing clones were selected. The hEREG gene is selected using Zeocin. Hepa1-6/hEREG cells were maintained and passaged in D-MEM (high glucose) medium (SIGMA) containing 10%FBS (SIGMA) and 400 μg/mL Zeocin.

(3-2) Evaluation by ADCC Reporter Bioassay

For measurement of *in vitro* ADCC activity, hFcγRIIIaV ADCC Reporter Bioassay, Effector cells, Propagation Model (Promega) was used. As target cells, 10 μL of Hepa1-6/hEREG cells adjusted to $5 \times 10^5$ cells/mL with the culture medium was added to each well of a 384-well plate. The medium used was Assay Buffer (96% RPMI, 4% FBS). Next, the antibodies produced in Example 1 and EGL-G4d (heavy chain SEQ ID NO: 28, light chain SEQ ID NO: 4) as a negative control, which has the sequence of human IgG4, were each diluted with the assay buffer to give 11 serial dilutions at a common ratio of 10 starting from the final concentration of 1 μg/mL, and then 10 μL each was added. Finally, as an effector cell solution, 10 μL of hFcγRIIIaV-expressing Jurkat cells adjusted to $3 \times 10^6$ cells/mL with the medium was added, and 30 μL in total was mixed. The mixture was then allowed to stand in a 5% $CO_2$ incubator at 37°C overnight. The plate was then allowed to stand at room temperature for 15 minutes, and 30 μL of a Bio-Glo reagent was added to each well. For the Bio-Glo reagent, Bio-Glo Luciferase Assay System (Buffer and Substrate) was used. Subsequently, the luminescence of each well was measured using a plate reader.

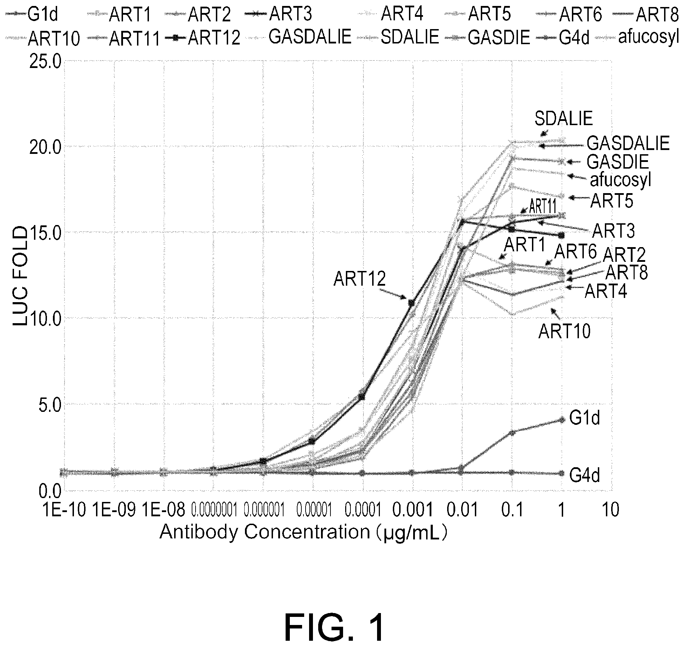

A fold induction value was determined by dividing the luminescence value of each well by the luminescence value of the well with no antibody added, and used as an index to evaluate the ADCC of each antibody. The obtained results are shown in Fig. 1. The EC 50 value of each sample was calculated using JMP 11.2.1 (SAS Institute Inc.), and is shown in Table 3.

(Table 3) EC50 value of hFcγRIIIaV-mediated reporter gene induction activity of each modified Fc-containing antibody

| Abbreviated name of antibody | EC50 value (μg/mL) |
|---|---|
| EGL-G1d | 4.63E-02 |
| EG L-ART1 | 7.16E-04 |
| EGL-ART2 | 1.35E-03 |
| EGL-ART3 | 1.53E-03 |
| EGL-ART4 | 7.89E-04 |
| EGL-ART5 | 1.67E-03 |
| EGL-ART6 | 1.50E-03 |
| EGL-ART8 | 1.07E-03 |
| EGL-ART10 | 1.07E-04 |
| EGL-ART11 | 4.13E-04 |
| EGL-ART12 | 3.04E-04 |
| EGL-GASDALIE | 2.08E-03 |
| EGL-SDALIE | 1.78E-03 |
| EGL-GASDIE | 3.70E-03 |
| EGL-G4d | N/A |
| EGL-afucosyl | 5.16E-03 |

These results showed that the antibodies with modified Fc produced this time exhibit a stronger reporter gene induction activity against Hepa1-6/hEREG cells than the wildtype human IgG1 constant region. The results of Table 3 also showed that all these variants exhibited the activity at lower concentrations than variants with symmetrically engineered CH2 domains and low-fucose antibodies produced by sugar chain modification. Among the variants produced this time, ART3, ART4, ART5, ART6, ART8, ART10, ART11, and ART12 were shown to have the activity at about the same or lower concentration than ART2. In particular, ART4, ART10, ART11, and ART12 exhibited the activity at lower concentrations even when compared to ART1, which has more enhanced hFcγRIIIaV binding than ART2.

[Example 4] Evaluation of antibodies having modified Fc regions by ADCP reporter bioassay

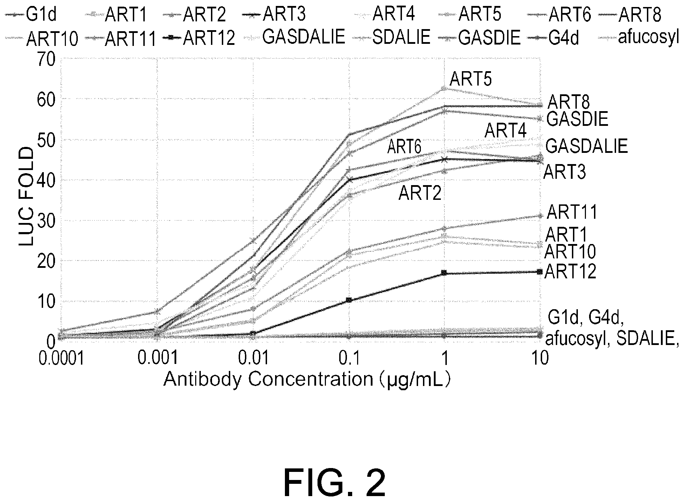

For measurement of *in vitro* ADCP activity, hFcyRIIaH ADCP Reporter Bioassay, Core Kit (Promega) was used. As target cells, 10 µL of Hepal-6/hEREG cells adjusted to 1 x 10$^6$ cells/mL with the culture medium was added to each well of a 384-well plate. Assay Buffer (96% RPMI, 4% FBS) was used as the culture medium. Next, the antibodies produced in Example 1 were each diluted with the assay buffer to final concentrations of 0, 0.001, 0.01, 0.1, 1, and 10 µg/mL, and then 10 µL each was added. Finally, as an effector cell solution, 10 µL of hFcyRIIaH-expressing Jurkat cells attached to the kit was added, and 30 µL in total was mixed. The mixture was then allowed to stand in a 5% CO$_2$ incubator at 37°C for 6 hours. The cell density of the hFcyRIIaH-expressing Jurkat cells was 9.68×10$^5$ cells/mL. The plate was then allowed to stand at room temperature for 15 minutes, and 30 µL of a Bio-Glo reagent was added to each well. For the Bio-Glo reagent, Bio-Glo Luciferase Assay System (Buffer and Substrate) was used. Subsequently, the luminescence of each well was measured using a plate reader. A fold induction value was determined by dividing the luminescence value of each well by the luminescence value of the well with no antibody added, and used as an index to evaluate the ADCP of each antibody. The obtained results are shown in Fig. 2. The EC 50 value of each sample was calculated using JMP 11.2.1 (SAS Institute Inc.), and is shown in Table 4.

(Table 4) EC50 value of hFcyRIIaH-mediated reporter gene induction activity of each modified Fc-containing antibody

| Abbreviated name of antibody | EC50 value (µg/mL) |
|---|---|
| EGL-G1d | N/A |
| EGL-ART1 | 3.53E-02 |
| EGL-ART2 | 2.18E-02 |
| EGL-ART3 | 1.58E-02 |
| EGL-ART4 | 4.32E-02 |
| EGL-ART5 | 2.45E-02 |
| EGL-ART6 | 1.92E-02 |
| EGL-ART8 | 1.71E-02 |
| EGL-ART10 | 4.19E-02 |
| EGL-ART11 | 3.67E-02 |
| EGL-ART12 | 8.50E-02 |
| EGL-GASDALIE | 3.24E-02 |
| EGL-SDALIE | N/A |
| EGL-GASDIE | 1.49E-02 |
| EGL-G4d | N/A |
| EGL-afucosyl | N/A |

These results showed that the antibodies with modified Fc produced this time exhibit a stronger reporter gene induction activity against Hepal-6/hEREG cells than the wildtype human IgG1 constant region. The results of Table 4 also showed that they exhibited the activity at lower concentrations than variants with symmetrically engineered CH2 domains and low-fucose antibodies produced by sugar chain modification. Moreover, among the variants produced this time, ART2, ART3, ART5, ART6, and ART8 were shown to have the activity at lower concentration than ART1. In particular, ART3, ART6, and ART8 exhibited the activity at lower concentrations even when compared to ART2, which has more enhanced hFcyRIIaH binding.

[Example 5] Evaluation of the antitumor effect of antibodies having modified Fc regions in a syngeneic tumor cell transplant model using human FcyR transgenic mice (5-1) Cell line

The Hepal-6/hEREG cells produced in Example 3-1 were maintained and passaged in D-MEM (high glucose) medium (SIGMA) containing 10%FBS (SIGMA) and 400 µg/mL Zeocin.

(5-2) Production of a syngeneic tumor line transplant mouse model

For the efficacy test, human FcγR transgenic mice (Proc Natl Acad Sci USA. 2012 Apr 17; 109(16): 6181-6186.) were used. Male mice at the age of 16 weeks were intraperitoneally given an anti-asialo GM1 antibody (aGM1, WAKO) at 100 μL/head in order to improve the cell engraftment rate. On the following day of aGM1 administration, a cell solution in which Hepal-6/hEREG cells and matrigel (CORNING) were mixed at 1:1 was subcutaneously given to transplant 1 × 10$^7$ cells. The model was determined to be established when the average volume of the grafted tumor reached approximately 300 mm$^3$ to 500 mm$^3$.
The volume of the grafted tumor was calculated with the following formula:

$$\text{Tumor volume} = \text{long diameter x short diameter x short diameter} /2$$

(5-3) Preparation of agents to be administered

EGL-ART6 produced in the present invention was expected to have the most potent antitumor activity in view of the A/I ratio results in Example 2 and the strength of the reporter gene induction activities in Examples 3 and 4. Thus, as the agents to be administered to the Hepal-6/hEREG cell transplant model, the anti-hEREG control antibody (EGL-Gld) and the anti-hEREG antibodies having Fc with enhanced FcγR binding (EGL-afucosyl and EGL-ART6) were produced by the same method as in Example 1, and each prepared at 1 mg/mL using His buffer (150 mM NaCl, 20 mM His-HCl buffer pH6.0).

(5-4) Agent administration in antitumor effect measurement

After 7 days of transplantation, EGL-Gld, EGL-afucosyl, and EGL-ART6 were administered at 10 mg/kg via the tail vein.
Details on the agent treatment in antitumor effect measurement are shown in Table 5.

(Table 5) Measurement of antitumor effect in Hepal-6/hEREG cell transplant model

| Group | Heads | Agent | Dose | Administration route | Administration date |
|---|---|---|---|---|---|
| 1 | 5 | His buffer | - | Tail vein | 7th day after transplantation |
| 2 | 5 | EGL-G1d | 10 mg/kg | | |
| 3 | 5 | EGL-afucosyl | 10 mg/kg | | |
| 4 | 5 | EGL-ART6 | 10 mg/kg | | |

(5-5) Evaluation of antitumor effect

Antitumor effect was evaluated by the tumor volume calculated using the formula shown in (5-2).
TGI (tumor growth inhibition) values were calculated using the following formula:

$$\text{TGI} = (1 - (\text{Average tumor volume of a group of interest at the time of measurement} - \text{Average tumor volume before antibody administration}) / (\text{Average tumor volume of the control group at the time of measurement} - \text{Average tumor volume before antibody administration})) \text{ x } 100$$

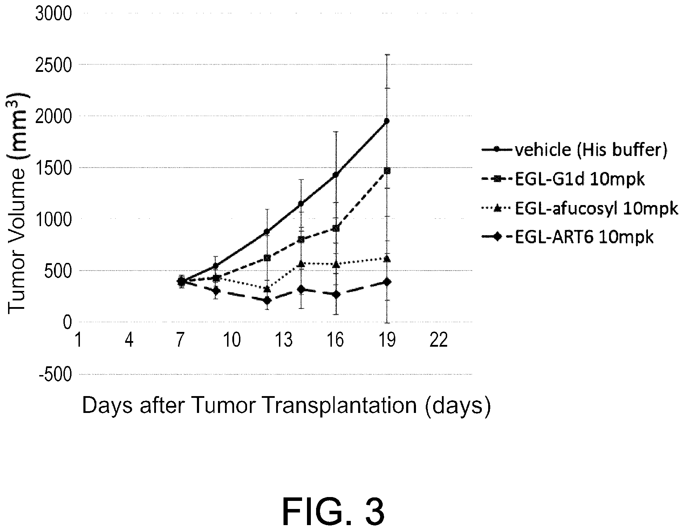

As a result, both FcγR binding-enhanced antibodies EGL-afucosyl and EGL-ART6, administered at 10 mg/kg, showed an efficacy of TGI=80 or higher on day 19 after administration. On the other hand, the control antibody EGL-G1d showed TGI=31 (Fig. 3). This confirmed that the *in vivo* antitumor effect of EGL-ART6 produced in the present invention is also enhanced as compared to EGL-afucosyl as expected.

[Example 6] Evaluation of C1q-binding activity of antibodies having modified Fc regions

While the CDC activity of antibodies has been reported to contribute to antitumor effects (Nat. Immunol., 2017, 18, 889), it is known to cause side effects derived from CDC activity, as represented by infusion-related reaction (J.

Immunol. 2008, 180, 2294-2298 and Br. J. Haematol. 2001, 115, 807-811). Therefore, even when developing antibody pharmaceuticals with enhanced ADCC activity and ADCP activity, it is preferred that the degree of CDC activity can be chosen depending on the target disease. The interaction between complement and Fc is mediated by C1q. According to reports of analyzing the interaction between C1q and Fc (Science, 2018, 359, 794-797 and Molecular Immunology 2012, 51, 66-72), the FcγR and C1q interaction sites on the Fc region appear to overlap in part. These articles report that the residues at EU numbering positions 329, 330, and 331 on Fc are important for interaction with C1q, and in addition, the residues at EU numbering positions 268, 270, and 298 also contribute to the binding with C1q. These positions and surrounding residues are modified in the present invention to enhance the FcγR binding. Therefore, the produced Fc region variants are likely to also have enhanced or reduced binding to C1q, and hence it may be possible to control CDC activity in developing antibody pharmaceuticals. Accordingly, the binding of the produced Fc region variants to C1q was evaluated.

The anti-human Epiregulin antibodies produced in Examples 1 and 3 were subjected to ELISA. In addition, the buffers shown in Table 6 were prepared as necessary. The antigen used was a human C1q protein (hClq).

(Table 6) Composition of buffers used in human C1q ELISA

| Buffer | Composition |
| --- | --- |
| Blocking/di lution buffer: | TBS, 0. 1% Tween20, 0. 5%BSA, 1x Block ace powder |
| Wash Buffer | PBST, pH7.4 |
| Stop Buffer | 0.5 mol/L sulfuric acid |

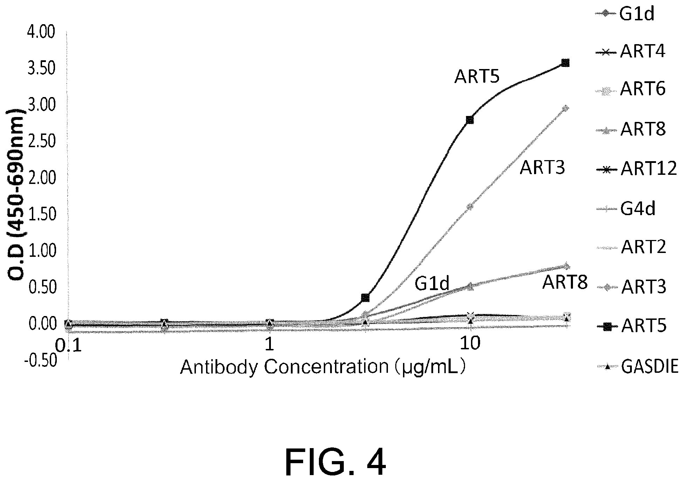

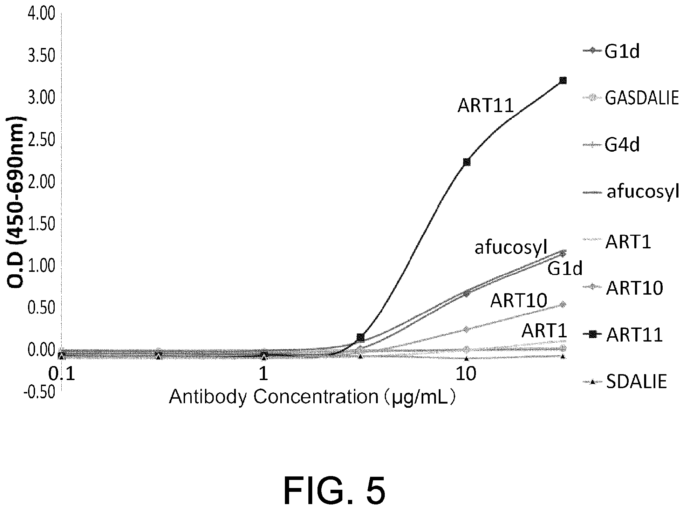

First, a 96-well maxisorp plate (Thermo fisher) was coated at 4°C overnight with 50 μL of solutions containing each antibody prepared at 30, 10, 3, 1, 0.3, 0.1, and 0.03 μg/mL in PBS. Each well of the plate was washed with Wash buffer to remove the antibody not bound to the plate, and then the wells were blocked with 200 μL of Blocking/dilution Buffer at room temperature for 2 hours or longer. After the Blocking/dilution Buffer was removed from each well, hClq (Calbiochem) prepared such that the final concentration was 3 μg/mL in Blocking/dilution Buffer was added at 50 μL per well. The plate was then allowed to stand at room temperature for 1 hour to allow hC1q to bind to the antibody within each well. After washing with Wash Buffer, 50 μL of an HRP-conjugated anti-hClq antibody (AbDSerotec) diluted with Blocking/dilution Buffer was added to each well, and the plate was incubated while standing for 1 hour. After washing with Wash Buffer, TMB single solution (Invitrogen) was added. The color development reaction of the solution in each well was stopped by addition of Stop Buffer, and then the developed color was measured by absorbance at 450 nm and 690 nm. The buffers used were those containing the composition shown in Table 6. The measured results are shown in Fig. 4 and Fig. 5.

As shown in Fig. 4 and Fig. 5, among the variants evaluated, ART3, ART5, and ART 11 had an enhanced C1q binding ability as compared to G1d. The binding ability of Afucosyl and ART8 was comparable to that of G1d. In addition, the C1q binding of ART 1, ART2, ART4, ART6, ART10, ART12, GASDALIE, SDALIE, and GASDIE was reduced as compared to that of Gld. Of these, the C1q binding ability of ART1, ART2, ART4, ART6, ART12, GASDALIE, SDALIE, and GASDIE was reduced to the same level as that of G4d, which has a human IgG4 sequence. As human IgG4 is considered to have little CDC activity (J. Immunol. Methods 2005, 306, 151-160), these variants whose binding to C1q is reduced to the same level as that of G4d would have little CDC activity like IgG4. The common amino acid modifications shared by these variants with greatly reduced C1q binding include a modification at Ala330 or a modification at Ile332. These regions, in particular Ala330, are very important regions for interaction with C1q. This gives the inference that it is the modification introduced into this site that greatly reduced the binding with C1q. On the other hand, ART3, ART5, and ART11, which had enhanced C1q binding as compared to Gld, did not have a modification introduced at position 330 or position 332, suggesting that the enhanced binding was due to the effect of the S298A modification or a modification at position 326, which is believed to improve the binding to C1q (Science, 2018, 359, 794-797).

Although the foregoing invention has been described in some detail by way of illustration and example for purposes of clarity of understanding, the descriptions and examples herein should not be construed as limiting the scope of the invention. The disclosures of all patent and scientific literature cited herein are expressly incorporated in their entirety by reference.

[Industrial Applicability]

The invention of the present disclosure provides polypeptides containing an Fc region variant that binds to activating FcγRIIa and FcγRIIIa more strongly and exhibits reduced binding to inhibitory FcγRIIb. The polypeptides of the present disclosure display high ADCC/ADCP activity and are useful in antitumor therapy (for example, treatment and/or prevention of inflammatory diseases, treatment and/or prevention of various cancers, etc.).

**Claims**

1. A polypeptide which comprises a variant Fc region comprising amino acid alterations in a parent Fc region, wherein the parent Fc region is composed of two polypeptide chains, and wherein the variant Fc region comprises amino acid alterations at the following positions:

    (i) positions 234, 235, 236, 239, 268, 270, and 298 according to EU numbering in the first polypeptide of the parent Fc region; and
    (ii) positions 270, 298, 326, and 334 according to EU numbering in the second polypeptide of the parent Fc region.

2. The polypeptide of claim 1, wherein the variant Fc region further comprises an amino acid alteration at position 326 according to EU numbering in the first polypeptide of the parent Fc region.

3. The polypeptide of claim 1 or 2, wherein the variant Fc region further comprises an amino acid alteration at position 236 according to EU numbering in the second polypeptide of the parent Fc region.

4. A polypeptide which comprises a variant Fc region comprising amino acid alterations in a parent Fc region, wherein the parent Fc region is composed of two polypeptide chains, and wherein the variant Fc region comprises amino acid alterations at the following positions:

    (i) positions 234, 235, 236, 239, 268, 270, 298, and 326 according to EU numbering in the first polypeptide of the parent Fc region; and
    (ii) positions 236, 270, 298, 326, and 334 according to EU numbering in the second polypeptide of the parent Fc region.

5. The polypeptide of any one of claims 1 to 4, wherein the variant Fc region further comprises an amino acid alteration at position 332 according to EU numbering in the first polypeptide of the parent Fc region.

6. The polypeptide of any one of claims 1 to 5, wherein the variant Fc region further comprises an amino acid alteration at position 330 according to EU numbering in the first polypeptide of the parent Fc region.

7. The polypeptide of any one of claims 1 to 6, wherein the variant Fc region further comprises an amino acid alteration at position 332 according to EU numbering in the second polypeptide of the parent Fc region.

8. The polypeptide of any one of claims 1 to 7, wherein the variant Fc region further comprises an amino acid alteration at position 330 according to EU numbering in the second polypeptide of the parent Fc region.

9. A polypeptide which comprises a variant Fc region comprising amino acid alterations in a parent Fc region, wherein the parent Fc region is composed of two polypeptide chains, and wherein the variant Fc region comprises amino acid alterations at the following positions:

    (i) positions 234, 235, 236, 239, 268, 270, 298, 330, and 332 according to EU numbering in the first polypeptide of the parent Fc region; and
    (ii) positions 236, 270, 298, 326, 330, 332, and 334 according to EU numbering in the second polypeptide of the parent Fc region.

10. The polypeptide of any one of claims 1 to 9, wherein the variant Fc region further comprises amino acid alterations at positions 250 and 307 according to EU numbering in the first polypeptide of the parent Fc region.

11. The polypeptide of any one of claims 1 to 10, wherein the variant Fc region further comprises amino acid alterations at positions 250 and 307 according to EU numbering in the second polypeptide of the parent Fc region.

12. The polypeptide of any one of claims 1 to 11, which comprises at least one amino acid alteration selected from the following amino acid alterations:

(i) Tyr or Phe at position 234, Gin or Tyr at position 235, Trp at position 236, Met at position 239, Val at position 250, Asp at position 268, Glu at position 270, Ala at position 298, Pro at position 307, Asp at position 326, Met at position 330, and Glu at position 332 according to EU numbering in the first polypeptide of the parent Fc region; and

(ii) Ala at position 236, Val at position 250, Glu at position 270, Ala at position 298, Pro at position 307, Asp at position 326, Met or Lys at position 330, Asp or Glu at position 332, and Glu at position 334 according to EU numbering in the second polypeptide of the parent Fc region.

13. The polypeptide of any one of claims 1 to 12, wherein binding activity to at least one Fcγ receptor selected from the group consisting of FcγRIa, FcγRIIa, FcγRIIb, and FcγRIIIa is enhanced in the variant Fc region as compared to the parent Fc region.

14. The polypeptide of any one of claims 1 to 13, wherein selectivity between an activating Fcγ receptor and an inhibitory Fcγ receptor is improved in the variant Fc region as compared to the parent Fc region.

15. The polypeptide of any one of claims 1 to 14, wherein the polypeptide comprising the variant Fc region is an antibody.

FIG. 1

FIG. 2

FIG. 3

FIG. 4

FIG. 5

## INTERNATIONAL SEARCH REPORT

| International application No. |
|---|
| PCT/JP2021/031448 |

| A. CLASSIFICATION OF SUBJECT MATTER |
|---|
| C07K 16/00(2006.01)i; C07K 16/46(2006.01)i; C12N 15/13(2006.01)i<br>FI: C07K16/00 ZNA; C12N15/13; C07K16/46 |
| According to International Patent Classification (IPC) or to both national classification and IPC |

| B. FIELDS SEARCHED |
|---|
| Minimum documentation searched (classification system followed by classification symbols)<br>C07K16/00; C07K16/46; C12N15/13 |

| Documentation searched other than minimum documentation to the extent that such documents are included in the fields searched |
|---|
| Published examined utility model applications of Japan     1922-1996<br>Published unexamined utility model applications of Japan     1971-2021<br>Registered utility model specifications of Japan     1996-2021<br>Published registered utility model applications of Japan     1994-2021 |

| Electronic data base consulted during the international search (name of data base and, where practicable, search terms used)<br>JSTPlus/JMEDPlus/JST7580 (JDreamIII); CAplus/MEDLINE/EMBASE/BIOSIS (STN);<br>SwissProt/GeneSeq |
|---|

| C. DOCUMENTS CONSIDERED TO BE RELEVANT |

| Category* | Citation of document, with indication, where appropriate, of the relevant passages | Relevant to claim No. |
|---|---|---|
| X | WO 2013/002362 A1 (CHUGAI PHARMACEUTICAL CO., LTD.) 03 January 2013 (2013-01-03) claims, examples 8, 24-25 | 1-15 |
| X | WO 2014/104165 A1 (CHUGAI PHARMACEUTICAL CO., LTD.) 03 July 2014 (2014-07-03) claims, examples 8, 24-25 | 1-15 |
| A | JP 2014-504265 A (ZYMEWORKS INC.) 20 February 2014 (2014-02-20) claims | 1-15 |
| A | JP 2014-509857 A (AMGEN INC.) 24 April 2014 (2014-04-24) claims | 1-15 |
| A | ESCOBAR-CABRERA, Eric et al., "Asymmetric Fc Engineering for Bispecific Antibodies with Reduced Effector Function", Antibodies, 16 May 2017, vol. 6, no. 7, pp. 1-16 entire text | 1-15 |

| ☒ | Further documents are listed in the continuation of Box C. | ☒ | See patent family annex. |
|---|---|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>10 September 2021 (10.09.2021) | Date of mailing of the international search report<br>21 September 2021 (21.09.2021) |
|---|---|
| Name and mailing address of the ISA/<br>  Japan Patent Office<br>  3-4-3, Kasumigaseki, Chiyoda-ku,<br>  Tokyo 100-8915, Japan | Authorized officer<br><br><br>Telephone No. |

Form PCT/ISA/210 (second sheet) (January 2015)

| | **INTERNATIONAL SEARCH REPORT** | International application No.<br>PCT/JP2021/031448 |
|---|---|---|

C (Continuation). DOCUMENTS CONSIDERED TO BE RELEVANT

| Category* | Citation of document, with indication, where appropriate, of the relevant passages | Relevant to claim No. |
|---|---|---|
| A | LIU, Zhi et al., "Asymmetrical Fe Engineering Greatly Enhances Antibody-dependent Cellular Cytotoxicity (ADCC) Effector Function and Stability of the Modified Antibodies", THE JOURNAL OF BIOLOGICAL CHEMISTRY, 05 December 2013, vol. 289, no. 6, pp. 3571-3590 entire text | 1-15 |

Form PCT/ISA/210 (continuation of second sheet) (January 2015)

**INTERNATIONAL SEARCH REPORT**
Information on patent family members

International application No.

PCT/JP2021/031448

| Patent Documents referred in the Report | Publication Date | Patent Family | Publication Date |
|---|---|---|---|
| WO 2013/002362 A1 | 03 Jan. 2013 | US 2014/0199294 A1 claims, examples 8, 24-25 EP 2728002 A1 | |
| WO 2014/104165 A1 | 03 Jul. 2014 | US 2015/0344570 A1 claims, examples 8, 24-25 EP 2940135 A1 | |
| JP 2014-504265 A | 20 Feb. 2014 | US 2012/0149876 A1 claims EP 2635607 A1 | |
| JP 2014-509857 A | 24 Apr. 2014 | US 2014/0112926 A1 claims EP 2686345 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2000042072 A
- WO 2006019447 A
- WO 2012058768 A
- WO 2012125850 A
- WO 2013002362 A
- WO 2014104165 A
- WO 200492219 A, Hinton
- US 5500362 A
- US 5821337 A
- US 6737056 B, Presta
- WO 2013118858 A
- WO 2006106905 A
- WO 1996027011 A
- WO 2016125495 A
- WO 2006053301 A
- WO 2009086320 A
- WO 2009041613 A
- WO 2011122011 A
- WO 2012132067 A
- WO 2013046704 A
- WO 2013180201 A
- WO 2013180200 A
- WO 2009125825 A
- WO 2012073992 A
- WO 2013047752 A
- EP 1752471 A
- EP 1772465 A
- WO 2012016227 A
- WO 2014145159 A
- US 5731168 A
- WO 2009089004 A1
- WO 2011028952 A
- WO 2008119353 A
- WO 2011131746 A
- WO 2007114325 A
- WO 1998050431 A
- WO 1995033844 A
- US 5959177 A
- US 6040498 A
- US 6420548 B
- US 7125978 B
- US 6417429 B
- WO 2006067913 A

**Non-patent literature cited in the description**

- *Nature Biotechnology,* 2005, vol. 23, 1073-1078
- *Eur. J. Pharm. Biopharm,* 2005, vol. 59 (3), 389-96
- *Chemical Immunology,* 1997, vol. 65, 88
- *Cancer Res.,* 2008, vol. 68, 8049-8057
- *Blood,* 2009, vol. 113, 3735-3743
- *Immunol. Lett.,* 2002, vol. 82, 57-65
- *Pro. Nat. Acad. Sci.,* 1998, vol. 95, 652-656
- *Nature Medicine,* 2000, vol. 6, 443-446
- *Blood,* 2002, vol. 99, 754-758
- *Ann. Oncol.,* 2011, vol. 22, 1302-1307
- *Science,* 2005, vol. 310, 1510-1512
- *Eur. J. Immunol.,* 1993, vol. 23, 1098
- *Immunology,* 1995, vol. 86, 319
- *Pro. Nat. Acad. Sci.,* 2006, vol. 103, 4005-4010
- *J. Biol. Chem.,* 2003, vol. 278, 3466-3473
- *J. Biol. Chem.,* 2001, vol. 276, 16469-16477
- *JCI Insight,* 2019, vol. 4, e131882
- *Mol. Cancer Ther.,* 2008, vol. 7, 2517-2527
- *Protein Eng. Des. Sel.,* 2013, vol. 26, 589-598
- **SAMBROOK et al.** Molecular Cloning: A Laboratory Manual. Cold Spring Harbor Laboratory Press, 2001
- Current Protocols in Molecular Biology. 2003
- Methods in Enzymology. Academic Press, Inc,
- PCR 2: A Practical Approach. 1995
- Antibodies, A Laboratory Manual. 1988
- Animal Cell Culture. 1987
- Oligonucleotide Synthesis. 1984
- Methods in Molecular Biology. Humana Press
- Cell Biology: A Laboratory Notebook. Academic Press, 1998
- **J. P. MATHER ; P.E. ROBERTS.** Introduction to Cell and Tissue Culture. Plenum Press, 1998
- Cell and Tissue Culture: Laboratory Procedures. J. Wiley and Sons, 1993
- Handbook of Experimental Immunology
- Gene Transfer Vectors for Mammalian Cells. 1987
- PCR: The Polymerase Chain Reaction. 1994
- Current Protocols in Immunology. 1991

- Short Protocols in Molecular Biology. Wiley and Sons, 1999
- **C.A. JANEWAY ; P. TRAVERS.** Immunobiology. 1997
- **P. FINCH.** *Antibodies,* 1997
- Antibodies: A Practical Approach. IRL Press, 1988
- Monoclonal Antibodies: A Practical Approach. Oxford University Press, 2000
- **E. HARLOW ; D. LANE.** Using Antibodies: A Laboratory Manual. Cold Spring Harbor Laboratory Press, 1999
- The Antibodies. Harwood Academic Publishers, 1995
- Cancer: Principles and Practice of Oncology. J.B. Lippincott Company, 1993
- **SINGLETON et al.** Dictionary of Microbiology and Molecular Biology. J. Wiley & Sons, 1994
- **MARCH.** Advanced Organic Chemistry Reactions, Mechanisms and Structure. John Wiley & Sons, 1992
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **KABAT et al.** Sequences of Proteins of Immunological Interest. National Institutes of Health, 1991
- **DAERON.** *Annu. Rev. Immunol.,* 1997, vol. 15, 203-234
- **RAVETCH ; KINET.** *Annu. Rev. Immunol,* 1991, vol. 9, 457-492
- **CAPEL et al.** *Immunomethods,* 1994, vol. 4, 25-34
- **DE HAAS et al.** *J. Lab. Clin. Med.,* 1995, vol. 126, 330-341
- **GUYER et al.** *J. Immunol.,* 1976, vol. 117, 587
- **KIM et al.** *J. Immunol.,* 1994, vol. 24, 249
- **GHETIE ; WARD.** *Immunol. Today,* 1997, vol. 18 (12), 592-598
- **GHETIE et al.** *Nature Biotechnology,* 1997, vol. 15 (7), 637-640
- **HINTON et al.** *J. Biol. Chem.,* 2004, vol. 279 (8), 6213-6216
- **RAVETCH ; KINET.** *Annu. Rev. Immunol,* 1991, vol. 9, 457-92
- **CLYNES et al.** *PNAS (USA),* 1998, vol. 95, 652-656
- Immunologic studies in humans. Current Protocols in Immunology. 1993
- **FLATMAN et al.** *J. Chromatogr. B,* 2007, vol. 848, 79-87
- *J Exp Med,* 1989, vol. 170, 1369-1385
- *Arthritis Rheum.,* 2003, vol. 48, 3242-3252
- **KONO et al.** *Hum. Mol. Genet.,* 2005, vol. 14, 2881-2892
- **KYOGOJU et al.** *Arthritis Rheum.,* 2002, vol. 46, 1242-1254
- **WARRMERDAM.** *J. Exp. Med.,* 1990, vol. 172, 19-25
- **GHETIE et al.** *Immunol. Today,* 1997, vol. 18 (12), 592-598
- **RAGHAVAN et al.** *Immunity,* 1994, vol. 1, 303-315
- **HINTON et al.** *J. Immunol.,* 2006, vol. 176 (1), 346-356
- **DALL'ACQUA et al.** *J. Biol. Chem.,* 2006, vol. 281 (33), 23514-23524
- **PETKOVA et al.** *Intl. Immunol.,* 2006, vol. 18 (12), 1759-1769
- **ZALEVSKY et al.** *Nat. Biotechnol.,* 2010, vol. 28 (2), 157-159
- *J. Biol. Chem.,* 2010, (285), 19637-19646
- **RIDGWAY et al.** *Prot. Eng.,* 1996, vol. 9, 617-621
- **MERCHANT et al.** *Nat.Biotech.,* 1998, vol. 16, 677-681
- **DAVIS et al.** *Prot. Eng. Des. & Sel.,* 2010, vol. 23, 195-202
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- **MATHER.** *Biol. Reprod.,* 1980, vol. 23, 243-251
- **MATHER et al.** *Annals N.Y. Acad. Sci.,* 1982, vol. 383, 44-68
- **URLAUB et al.** *Proc. Natl. Acad. Sci. USA,* 1980, vol. 77, 4216
- **LAZAR et al.** *Proc. Natl. Acad. Sci. USA,* 2006, vol. 103 (11), 4005-4010
- *Nat. Biotechnol.,* 1998, vol. 16, 677
- *Protein Science,* 1995, vol. 4, 2411-2423
- *Glycobiol.,* 2006, vol. 17 (1), 104-118
- *Biotechnol, Bioeng.,* 2006, vol. 93 (5), 851-861
- *Nature Medicine,* 2000, vol. 6, 443-436
- *Proc Natl Acad Sci USA.,* 17 April 2012, vol. 109 (16), 6181-6186
- *Nat. Immunol.,* 2017, vol. 18, 889
- *J. Immunol.,* 2008, vol. 180, 2294-2298
- *Br. J. Haematol.,* 2001, vol. 115, 807-811
- *Science,* 2018, vol. 359, 794-797
- *Molecular Immunology,* 2012, vol. 51, 66-72
- *J. Immunol. Methods,* 2005, vol. 306, 151-160